# EUROPEAN PATENT SPECIFICATION

(11) **EP 4 637 868 B1**
(45) Date of publication and mention of the grant of the patent: **07.10.2026**
(21) Application number: 23834107.7
(22) Date of filing: 19.12.2023
(51) Int. Cl.: A61M 1/16, A61M 1/28, A61M 5/14, B01F 23/00

(54) **MIXING SYSTEMS FOR IN-LINE MIXING OF COMPONENTS OF A MEDICAL FLUID**
MISCHSYSTEME ZUM INLINE-MISCHEN VON KOMPONENTEN EINER MEDIZINISCHEN FLÜSSIGKEIT
SYSTÈMES DE MÉLANGE POUR LE MÉLANGE EN LIGNE DE COMPOSANTS D'UN FLUIDE MÉDICAL

(30) Priority: 20.12.2022 EP 22214982
(43) Date of publication of application: 29.10.2025
(73) Proprietor: Gambro Lundia AB, 226 43 Lund (SE)
(72) Inventor: JANSSON, Olof, 235 38 Vellinge (SE); VARTIA, Christian, 247 64 Veberöd (SE)
(74) Representative: Sweden SHS IP Office
(86) International application number: PCT/EP2023/086516
(87) International publication number: WO 2024/133180

(56) References cited:
- EP-A1- 0 503 047
- US-A- 5 762 769
- US-A1- 2019 262 526

## Description

### Technical field

The present disclosure relates generally to dialysis therapy, and in particular to mixing medical fluid to be used in the dialysis therapy from concentrates and pure water.

### Background

Dialysis therapy is used for treating individuals suffering from acute or chronic renal insufficiency. Dialysis removes waste, toxins and excess water from the body that normal functioning kidneys would otherwise remove.

One type of dialysis therapy is extracorporeal (EC) blood therapy, in which blood from a patient is pumped through an EC blood circuit back to the patient. A blood filtration unit, commonly known as a dialyzer, is arranged in the EC blood circuit to interface the blood with a dialysis fluid over a semi-permeable membrane. One modality of EC blood therapy is hemodialysis (HD) which in general uses diffusion to remove waste products from the blood. A diffusive gradient occurs across the semi-permeable membrane and the dialysis fluid. Another modality is hemofiltration (HF), which relies on convective transport of toxins from the patient's blood. HF is accomplished by adding another dialysis fluid, referred to as infusion fluid, substitution fluid or replacement fluid, to the extracorporeal blood circuit during dialysis therapy. The replacement fluid and excess fluid accumulated by the patient in between therapy sessions is ultrafiltered over the course of HF therapy, providing a convective transport mechanism that is particularly beneficial in removing middle and large molecules. Yet another modality is hemodiafiltration (HDF), which combines convective and diffusive clearances. HDF uses dialysis fluid flowing through a dialyzer, similar to standard hemodialysis, to provide diffusive clearance. In addition, replacement fluid is delivered directly to the extracorporeal blood circuit, providing convective clearance. Here, more fluid than the patient's excess fluid is removed from the blood, causing the increased convective transport of waste products from the blood. The additional fluid removed is replaced via the replacement fluid. EC blood therapy may be employed as an intensive care (IC) treatment, e.g., as Continuous Renal replacement Therapy (CRRT), or as Intermittent hemodialysis (IHD), with variants such as Sustained Low Efficiency Dialysis/Sustained Low Efficiency Daily Dialysis (SLED/SLEDD) and Extended Daily Dialysis (EDD).

Another type of dialysis therapy is peritoneal dialysis (PD), in which a dialysis fluid is infused into a patient's peritoneal cavity. The dialysis fluid is in contact with the peritoneal membrane located in the patient's peritoneal cavity. Waste, toxins and excess water pass from the patient's bloodstream, through the capillaries in the peritoneal membrane, and into the dialysis fluid by diffusion and osmosis, i.e., an osmotic gradient occurs across the membrane. An osmotic agent in the dialysis fluid provides the osmotic gradient. Used or spent dialysis fluid is drained from the patient, removing waste, toxins and excess water from the patient. This cycle is repeated, for example multiple times.

There are various types of PD therapies, including continuous ambulatory PD (CAPD), automated PD (APD), tidal PD (TPD), and continuous flow PD (CFPD). CAPD is a manual dialysis treatment, in which the flow of dialysis fluid into and out of the patient is driven by gravity. APD is performed by a dialysis machine, commonly known as a cycler, which is fluidly connected to the peritoneal cavity and operated to automatically transfer dialysis fluid to and from the peritoneal cavity in accordance with a predefined schedule, for example during the night while the patient is sleeping. TPD is a type of automated PD where, after an initial complete fill, only a part of the filling volume in the peritoneal cavity is exchanged. CFPD requires two catheters, or a double lumen catheter, to, after an initial fill, maintain a continuous flow of dialysis fluid at a high flow rate into an out of the peritoneal cavity.

Conventionally, dialysis fluid for PD is delivered in pre-filled bags to the point-of-care, for example an intensive care (IC) unit or the home of the patient. EC blood therapy may also use pre-filled bags of dialysis fluid, for example in an IC unit, for treatment of acute kidney failure. Dialysis fluid for EC blood treatment of chronic kidney failure is typically produced by the dialysis machine itself, by mixing one or more concentrates with pure water. Recently, dialysis machines that produce dialysis fluid for PD have also been made commercially available. Hereinafter, dialysis fluid, infusion fluid, replacement fluid and substitution fluid are referred to as medical fluids.

Production of medical fluids at the point of use is attractive since it reduces the cost and environmental impact of transporting large amounts of ready-made medical fluid and the burden of storing and handling pre-filled bags. Production of medical fluid requires access to pure water and concentrates. Typically, a water purifier is connected to a tap water source, and a fluid generation unit is operated to mix one or more concentrates with the pure water to generate the medical fluid. It is important that the final mixed medical fluid reaches a predetermined composition, including intended concentrations of the one or more concentrates. Concentration is typically monitored using one or more concentration sensors, such as, e.g., one or more conductivity sensors. Some concentrates' conductivity contribution is difficult to measure as they are dosed in low amounts and gives a low or no conductivity response, such as glucose and potassium. Such dosing may instead rely on volumetric dosing where the concentration of the concentrates in the bags is assumed to be correct. However, evaporation from the bags and variations according to manufacturing specifications may cause concentration fluctuations. Sometimes a plurality of conductivity sensors is used to enable sensing of conductivity in different ranges. One conductivity sensor can then e.g., sense in a low conductivity range, while another sensor can sense in another higher conductivity range. However, having many conductivity sensors add cost to the system, and it is desired to keep the number down. Also, it may be desired to use conductivity sensors for protective purposes, whereby they might need to be configured to measure in ranges that do not encompass the low conductivity range.

US5900136 proposes to measure a concentration of a nonelectrolyte, e.g., glucose, in an electrolyte solution based on a previously determined correlation between the electric conductivity and the concentration of the nonelectrolyte in the same system. Data for determining the correlation must then be determined in beforehand.

US2019/0262526A1 suggests adding an electrolyte concentrate marker to an osmotic agent concentrate or glucose concentrate to permit the concentration of the osmotic agent to be inferred from a measurement of the diluted agent. However, prior knowledge of the ratio of osmotic agent concentrate to electrolyte concentrate marker must be known.

Hence, there is room for improvement for providing an accurately mixed treatment solution.

### Summary

It is an objective of the disclosure to alleviate at least some of the drawbacks with the prior art. It is a further objective to provide techniques for accurate dosing of components that give small signal responses when dosed in their intended dosing rates, using concentration feedback. It is a further objective to provide techniques for accurate mixing of fluid to be used in dialysis.

These objectives and others are at least partly achieved by the method and mixing system according to the independent claim 1, and by the embodiments according to the dependent claims.

According to one aspect, the disclosure relates to a method for in-line mixing of components of a medical fluid in a mixing system, the medical fluid having a final predetermined composition of pure water, an SSR-component (a small-signal-response component), and optionally at least one electrolyte component. The method comprises: providing S1 a flow of fluid comprising pure water, or a mixture of pure water and an electrolyte component, in a main fluid line, and monitoring S2, with a concentration sensor, a concentration of the fluid. The method further comprises providing S3, with an SSR dosing mechanism, the SSR-component into the flow of fluid upstream the concentration sensor. The method further comprises controlling S4, with the SSR dosing mechanism, the dosing rate of the SSR-component to an initial dosing rate at which the concentration monitored with the concentration sensor indicates an initial concentration *c_{SSR_init}* of the SSR-component in the fluid that is greater than an intended final predetermined concentration *c_{SSR_final}* of the SSR-component in the medical fluid, in order to determine a relationship between the dosing rate of the SSR-component and the resulting concentration of the SSR-component in the fluid. The method further comprises downscaling S5, with the SSR dosing mechanism, the dosing rate of the SSR-component to a final dosing rate *Q_{SSR_final}* at which the final predetermined concentration *C_{SSR}__{final}* of the SSR-component in the fluid is achieved, based on the determined relationship.

Composition control using concentration feedback is beneficial as it becomes possible to take concentrate errors into account, such as concentration fluctuation because of evaporation or manufacturing deviations. Small Signal Response (SSR) component composition control has been identified to be challenging due to the weak or small concentration response of the SSR-component. The invention solves this problem by temporarily overdosing the SSR-component, whereby its contribution to the concentration is increased such that is becomes measurable within desired limits. By increasing the concentration of the SSR-component prior to establishing the final solution, impact on dosing accuracy from signal variations due to measuring noise and flow variation noise is minimized. This reduces the negative effect of the noise factors and therefore the desired concentration of SSR-components can be more accurately determined. The method is easy to perform and makes mixing more accurate.

According to some embodiments, the initial concentration *c_{SSR_init}* of the SSR-component in the fluid is greater than the intended final predetermined concentration *c_{SSR_final}* of the SSR-component in the medical fluid such that a ratio between a concentration response resulting from SSR-component dosing at the initial dosing rate *Q_{SSR_init}* and a concentration signal noise is equal to or greater than a predetermined limit.

According to some embodiments, the initial dosing rate *Q_{SSR_init}* of the SSR-component is > 1 to 20 times larger than the final dosing rate *Q_{SSR_final}.*

According to some embodiments, the SSR-component, being either of a non-conductive solution or a conductive solution such that, when added to the flow of fluid in the main flow path at an intended final dosing rate *Q_{SSR_final}* at which the final predetermined concentration *c_{SSR_init}* of the SSR-component in the fluid is achieved, its contribution to the concentration as monitored with the concentration sensor is too small to be measured with sufficient accuracy.

According to some embodiments, the concentration measured with the concentration sensor at the initial dosing rate corresponds to a predetermined target concentration κ*_{SSR_init}* of the fluid.

According to some embodiments, the initial dosing rate corresponds to a predetermined initial dosing rate *Q_{SSR_init}.*

According to some embodiments, the controlling S4 comprises determining the final dosing rate *Q*_{*SSR*_*final*} as being equal to a ratio between a final value *F_{final}* correlated with the intended final predetermined concentration *c_{SSR_final}* of the SSR-component and an initial value *Fᵢₙᵢₜᵢₐₗ* correlated with the initial concentration *c_{SSR_init}* of the SSR-component, multiplied with the initial dosing rate *Q_{SSR_init}.*

According to some embodiments, the providing S1 a flow of fluid comprises controlling a main flow rate of the fluid flow to be a predetermined fluid rate of medical fluid.

According to some embodiments, the predetermined fluid flow rate is a flow rate of medical fluid configured for a downstream located device or user.

According to some embodiments, the providing S1 a flow of fluid comprises controlling the main flow rate using a main pump arranged to the main fluid line.

According to some embodiments, the downscaling S5 comprises fixing a ratio between the final dosing rate *Q_{SSR_final}* of the SSR-component and the main flow rate.

According to some embodiments, the concentration sensor is a conductivity sensor.

According to some embodiments, the concentration sensor has a measuring range of 0.1 to 50 mS/cm, more preferably of 5 to 20 mS/cm.

According to some embodiments, the monitoring S2 is performed with the same concentration sensor.

According to some embodiments, the providing S1 a flow of fluid comprises providing the electrolyte component, with an electrolyte component dosing mechanism, at a dosing rate into the flow of pure water in the main fluid line to form a flow of fluid comprising a mixture of pure water and the electrolyte component upstream the concentration sensor.

According to some embodiments, the providing S1 a flow of fluid comprises, preceding the providing of the SSR-component into the flow of fluid, controlling the dosing rate of the electrolyte component, with the electrolyte component dosing mechanism, to a final dosing rate *Q_{A_final}* providing a final predetermined concentration *c_{A_final}* of the electrolyte component in the final medical fluid, based on the concentration monitored by the concentration sensor of the mixture of pure water and the electrolyte component.

According to some embodiments, the providing S1 a flow of fluid comprises fixing a ratio between the final dosing rate *Q_{A_final}* of the electrolyte component and the main flow rate.

According to some embodiments, the SSR-component decreases the conductivity of the fluid into which it is added.

According to some embodiments, the SSR-component comprises glucose.

According to some embodiments, the SSR-component is a liquid glucose concentrate comprising between 40-75% of glucose.

According to some embodiments, the initial concentration *c_{SSR_init}* of the SSR-component in the fluid is 4 to 20 percent, more preferably between 4 and 10 percent.

According to some embodiments, the SSR-component increases the conductivity of the fluid into which it is added.

According to some embodiments, the SSR-component comprises potassium.

According to some embodiments, the SSR-component comprises potassium with the concentration of 400-3200 mmol/l.

According to some embodiments, comprising providing S6 an additional electrolyte component, with an additional electrolyte component dosing mechanism, into the flow of fluid in the main fluid line to form a mixture of pure water, the electrolyte component, the SSR-component and the additional electrolyte component.

According to some embodiments, wherein the providing S6 comprises controlling the dosing rate of the additional electrolyte component, with the additional electrolyte component dosing mechanism, to a final dosing rate *Q_{B_final}* providing a final predetermined concentration *c_{B_final}* of the additional electrolyte component in the final predetermined composition of the medical fluid, based on the concentration monitored by the concentration sensor of the mixture of pure water, the electrolyte component and the additional electrolyte component.

According to some embodiments, the providing S1 a flow of fluid comprises providing an additional electrolyte component, with an additional electrolyte component dosing mechanism, at a dosing rate into the flow of pure water in the main fluid line to form a flow of fluid comprising a mixture of pure water, the electrolyte component and the additional electrolyte component upstream the concentration sensor.

According to some embodiments, the providing S1 a flow of fluid comprises, preceding the providing of the SSR-component into the flow of fluid, controlling the dosing rate of the additional electrolyte component, with the additional electrolyte component dosing mechanism, to a final dosing rate *Q_{B_final}* providing a final predetermined concentration *c_{B_final}* of the additional electrolyte component in the final medical fluid, based on the concentration monitored by the concentration sensor of the mixture of pure water, the electrolyte component and the additional electrolyte component.

According to some embodiments, the concentration sensor is a glucose sensor configured to measure glucose concentration.

According to a second aspect, the disclosure relates to a mixing system for in-line mixing of components of a medical fluid having a final predetermined composition of pure water, an SSR-component (a small-signal-response component), and optionally at least one electrolyte component. The mixing system comprises a fluid path. The fluid path comprises a main fluid line arranged to be connected to a source of pure water, an SSR-component line fluidly connected to the main fluid line and provided with an SSR-component line connector configured to be connected to an SSR-component container, and optionally an electrolyte component line fluidly connected to the main fluid line and provided with an electrolyte component connector configured to be connected to an electrolyte component container. The mixing system further comprises a main pump arranged to the main fluid line to provide a main flow of fluid in the main fluid line, a concentration sensor arranged to measure a concentration of the fluid in the main fluid line, an SSR dosing mechanism arranged to the SSR-component line to provide the SSR-component at a dosing rate into the main fluid line upstream the concentration sensor, and optionally an electrolyte dosing mechanism arranged to the electrolyte component line to provide an electrolyte component at a dosing rate into the main fluid line upstream the concentration sensor. The mixing system further comprises a control arrangement configured to provide, using the main pump, a flow of fluid comprising pure water from the source of pure water, or a mixture of pure water from the source of pure water and the electrolyte component from the electrolyte component container, in the main fluid line, and monitor, using the concentration sensor, a concentration of the fluid. The control arrangement is further configured to control, using the SSR dosing mechanism, the dosing rate of the SSR-component to an initial dosing rate at which the concentration monitored with the concentration sensor indicates an initial concentration *c_{SSR_init}* of the SSR-component in the fluid that is greater than an intended final predetermined concentration *c_{SSR_final}* of the SSR-component in the medical fluid, in order to determine a relationship between the dosing rate of the SSR-component and the resulting concentration of the SSR-component in the fluid. The control arrangement is further configured to downscale, using the SSR dosing mechanism, the dosing rate of the SSR-component to a final dosing rate *Q_{SSR_final}* at which the final predetermined concentration *c_{SSR_final}* of the SSR-component in the fluid is achieved, based on the determined relationship.

According to some embodiments, the initial concentration *c_{SSR_init}* of the SSR-component in the fluid is greater than the intended final predetermined concentration *C_{SSR_final}* of the SSR-component in the medical fluid such that a ratio between a concentration response resulting from SSR-component dosing at the initial dosing rate *Q_{SSR_init}* and concentration signal noise is equal to or greater than a predetermined limit.

According to some embodiments, the initial dosing rate *Q_{SSR_init}* of the SSR-component is > 1 to 20 times larger than the final dosing rate *Q_{SSR_final}.*

According to some embodiments, the SSR-component being either of a non-conductive solution or a conductive solution such that, when added to the flow of fluid in the main flow path at an intended final dosing rate *Q_{SSR_final}* at which the final predetermined concentration *c_{SSR_final}* of the SSR-component in the fluid is achieved, its contribution to the concentration as monitored with the concentration sensor is too small to be measured with sufficient accuracy.

According to some embodiments, the concentration measured with the concentration sensor at the initial dosing rate *Q_{SSR_init}* corresponds to a predetermined target concentration κ*_{SSR_init}* of the fluid.

According to some embodiments, the initial dosing rate corresponds to a predetermined initial dosing rate *Q_{SSR_init}.*

According to some embodiments, the control arrangement is configured to determine the final dosing rate *Q_{SSR_final}* as being equal to a ratio between a final value *F_{final}* correlated with the intended final predetermined concentration *c_{SSR_final}* of the SSR-component and an initial value *Fᵢₙᵢₜᵢₐₗ* correlated with the initial concentration *c_{SSR_final}* of the SSR-component, multiplied with the initial dosing rate *Q_{SSR_init}.*

According to some embodiments, the control arrangement is configured to control the main pump to provide the main flow rate to be a predetermined fluid rate of medical fluid.

According to some embodiments, the predetermined fluid rate is a flow rate of medical fluid configured for a downstream located device or user.

According to some embodiments, the control arrangement is configured to fix a ratio between the final dosing rate *Q_{SSR_final}* of the SSR-component and the main flow rate.

According to some embodiments, the concentration sensor is a conductivity sensor.

According to some embodiments, the concentration sensor has a measuring range of 0.1 to 50 mS/cm, more preferably of 5 to 20 mS/cm.

According to some embodiments, the control arrangement is configured to monitor with the same concentration sensor.

According to some embodiments, the control arrangement is configured to provide the electrolyte component, with the electrolyte component dosing mechanism, at a dosing rate into the flow of pure water in the main fluid line to form a flow of fluid comprising a mixture of pure water and the electrolyte component upstream the concentration sensor.

According to some embodiments, the control arrangement is configured to, preceding the providing of the SSR-component into the flow of fluid, control the dosing rate of the electrolyte component, with the electrolyte component dosing mechanism, to a final dosing rate *Q_{A_final}* providing a final predetermined concentration *c_{A_final}* of the electrolyte component in the final medical fluid, based on the concentration monitored by the concentration sensor of the mixture of pure water and the electrolyte component.

According to some embodiments, the control arrangement is configured to fix a ratio between the final dosing rate *Q_{A_final}* of the electrolyte component and the main flow rate.

According to some embodiments, the SSR-component decreases the conductivity of the fluid into which it is added.

According to some embodiments, the SSR-component comprises glucose.

According to some embodiments, the SSR-component is a liquid glucose concentrate comprising between 40-75% of glucose.

According to some embodiments, the initial concentration *c_{SSR_init}* of the SSR-component in the fluid is 4 to 20 percent, more preferably between 4 and 10 percent.

According to some embodiments, the SSR-component increases the conductivity of the fluid into which it is added.

According to some embodiments, the SSR-component comprises potassium.

According to some embodiments, the SSR-component comprises potassium with the concentration of 400-3200 mmol/l.

According to some embodiments, the fluid path comprises an additional electrolyte component line fluidly connected to the main fluid line and provided with an additional electrolyte component connector configured to be connected to an additional electrolyte component container. The mixing system further comprises an additional electrolyte component dosing mechanism arranged to the additional electrolyte component line, wherein the control arrangement is configured to provide the additional electrolyte components with the additional electrolyte component dosing mechanism, at a dosing rate into the flow of fluid in the main fluid line to form a mixture of pure water, the electrolyte component, the SSR-component and the additional electrolyte component.

According to some embodiments, the control arrangement is configured to control the dosing rate of the additional electrolyte component, with the additional electrolyte component dosing mechanism, to a final dosing rate *Q_{B_final}* providing a final predetermined concentration *c_{B_final}* of the electrolyte component in the final predetermined composition of the medical fluid, based on the concentration monitored by the concentration sensor of the mixture of pure water, the electrolyte component, the SSR-component and the additional electrolyte component.

According to some embodiments, the mixing system comprises a waste line fluidly connected to the main line downstream the concentration sensor and the main pump.

According to some embodiments, the mixing system comprises one or more valves configured to be controlled by the control arrangement to direct fluid in the main line to waste or to an end point of the main line.

According to some embodiments, the concentration sensor is a glucose sensor configured to measure glucose concentration.

According to a third aspect, the disclosure relates to a computer program comprising instructions to cause a system according to the second aspect to execute the steps of the method according to the first aspect.

According to a fourth aspect, the disclosure relates to a computer-implemented medium having stored thereon the computer program of the third aspect.

### Brief description of the drawings

Fig. 1 is a schematic view of an example dialysis treatment system including a mixing system.
Fig. 2 is a schematic view of an example mixing system according to some embodiments.
Figs. 3A to 3C and 4A to 4D are illustrations of different steps for dosing a small signal response component in the system of Fig. 2 according to some embodiments of the disclosure.
Fig. 5 is a flow chart of example methods for mixing components of a medical fluid in the system of Fig. 2.
Fig. 6 is two diagrams illustrating an example of dosing of a small signal response component.
Fig. 7 is a schematic view of an example mixing system according to some embodiments.
Figs. 8A to 8E are illustrations of different steps for accurately dosing a small signal response component in the system of Fig. 7 according to some embodiments of the disclosure.
Fig. 9 is a flow chart of example methods for mixing components of a medical fluid in the system of Fig. 7.
Fig. 10 is a schematic view of an example mixing system according to some embodiments.
Figs. 11A to 11F are illustrations of different steps for accurately dosing a small signal response component in the system of Fig. 10 according to some embodiments of the disclosure.

### Detailed description

In the following description, different example mixing systems will be illustrated together with techniques for accomplishing accurate dosing of components in these mixing systems, in particular small signal response (SSR-) components, that when added, forms medical fluid or forms part of a medical fluid to be used in a medical apparatus or used in medical treatments, for example dialysis treatment. The underlying idea makes it possible to improve concentration accuracy of an SSR-component by using concentration feedback from a fluid into which the SSR-component is dosed. This is done by increasing the dosing rate of the SSR-component during an initial setup-phase, compared to an intended dosing rate of the SSR-component, such that the concentration contribution of the SSR-component in the fluid is increased and thereby the signal-to-noise relationship of the concentration signal becomes sufficient for control. Thereafter the dosing rate of the SSR-component is downscaled to achieve the intended (nominal) concentration of SSR-component in the medical fluid.

A medical fluid is here a mix of pure water at least one SSR-component, and in some embodiments also at least one electrolyte component. The medical fluid is for example a liquid to be used by the medical apparatus itself. Alternatively, the medical fluid may be an IV fluid (Intravenous Fluid), dialysis fluid, an infusion fluid, a replacement fluid, or a substitution fluid. Such medical fluid may also be referred to as a treatment fluid.

An SSR-component is defined to be a component that, when dosed in an intended dosage rate, to a fluid consisting of pure water, or a mixture of pure water and one or more electrolyte components of a medical fluid, to reach a nominal concentration of the component in the medical fluid, gives a small contribution to a concentration property in the fluid into which it is dosed. This means that the SSR-component will give a small signal response when measured with a concentration sensor configured to measure the concentration property in the fluid. The small signal response is so small that it is difficult to measure with desired accuracy using the desired concentration sensor.

A nominal concentration of a component is an intended final predetermined concentration of the component in a final medical fluid according to a prescription. The intended dosage rate of the SSR-component is typically small (less than 5%) in relation to the flow rate of the fluid into which the SSR-component is dosed. However, in some embodiments the intended dosage rate is greater, e.g., 5 to 30 %, and the SSR-component will still give a small signal response in the fluid into which it is dosed. Hence, one aspect is the nature of the concentrate, i.e., the SSR-component, in terms of its ability to create a sufficient signal when dosed under normal conditions, and its concentration in the concentrate. Generally, an SSR-component, when dosed to reach a nominal concentration in a final solution, gives a small contribution to a concentration property to be measured in the final solution.

The concentration property is for example glucose concentration or conductivity. Glucose concentration, or simply glucose, may for example be measured with a glucose sensor. The glucose sensor may measure viscosity of the fluid, e.g., via sound velocity measurement (m/s) or refractive index measurement. Conductivity is typically measured with a conductivity sensor, also referred to as a conductivity cell. Generally, conductivity and concentration are related, such that by measuring conductivity the concentration can be established by knowing the ionic composition of the fluid. A relation between conductivity and concentration may be determined as a function. The conductivity sensor may also include a temperature sensor, to compensate the sensed conductivity, as conductivity changes with temperature. Conductivity is a measure of the ability of a solution to conduct electric current, measured in S/m, mS/cm or µS/cm.

A small signal response is for example caused by a concentration between 1-5% (for glucose) or between 1-4 mmol/L (for potassium) when being a component of a dialysis fluid. Typically, a small signal response corresponds to a change in concentration, e.g., measured in conductivity or glucose concentration, of up to 3% (compared to a base level when the measurement starts).

More generally, the SSR-component may be either of a nonelectrolyte component or an electrolyte component that, when dosed to reach a nominal concentration of the component in the medical fluid, causes a small signal response. Alternatively, the SSR-component may itself be composed of a mixture of nonelectrolytes and electrolytes. However, in some embodiments, the mixture of nonelectrolytes and electrolytes should either increase or decrease the conductivity of the fluid to which it is added, in order to be measurable by a concentration sensor being a conductivity sensor. In one embodiment, the SSR-component is either of a non-conductive solution or a conductive solution such that, when added to a flow of fluid in a main flow path at an intended final dosing rate *Q_{SSR_final}* at which the final predetermined concentration *c_{SSR_final}* of the SSR-component in the fluid is achieved, its contribution to the concentration as monitored with a concentration sensor is too small to be measured with sufficient accuracy.

A nonelectrolyte component does not conduct electric current. A nonelectrolyte component includes one or more nonelectrolytes. A nonelectrolyte may e.g., be made from a solid substance that does not readily create (dissociate into) positive and negative ions when dissolved in a solvent. If a nonelectrolyte component is mixed into a solution comprising electrolytes, it will lower the ability of the ions to move, i.e., it decreases the solution's conductance. The nonelectrolyte component decreases the conductivity by increasing the solution's viscosity (decreased ion mobility). A nonelectrolyte is for example a monosaccharide such as glucose (C₆H₁₂O₆) (sometimes also referred to as dextrose) dissolved in water or Icodextrin (C₆H₁₀O₅) (derived from maltodextrin). Solutions being composed of combinations of these nonelectrolytes are also possible. Glucose/dextrose may for example be in anhydrous or monohydrate form. An example nonelectrolyte component for PD, considered herein as an SSR-component, is for example a liquid concentrate comprising between 40-75% of glucose, in some embodiments between 55% and 64% glucose, in some embodiments 50% or 59% glucose.

An electrolyte component conducts electric current. An electrolyte component includes one or more electrolytes. An electrolyte is made from a solid substance that create (dissociate into) positive and negative ions when dissolved in a solvent. In other words, the solid substance, such as a salt NaCl, when dissolved in a solvent, turns the solution into an electrolyte. An electrolyte component is for example an aqueous solution where free moving ions are present. An electrolyte component to be dosed in a low amount may be regarded as an SSR-component. For example, a solution comprising ions, from one or more dissolved salts, such as sodium (Na⁺), potassium (K⁺), calcium (Ca²⁺), magnesium (Mg²⁺), or chloride (Cl⁻), bicarbonate (HCO₃⁻), or citrate (C₆H₅O₇³⁻), or combinations thereof, and the like may be regarded as an SSR-component. An electrolyte component considered herein as an SSR-component is for example a liquid electrolyte concentrate comprising potassium chloride (KCl), calcium chloride (CaCl₂), magnesium chloride (MgCl₂), and an acid (as in Baxter's SelectBag^{™}). One product example of a SelectBag^{™} is: 200 mM/L KCl, 250 mM/L CaCl2, 100 mM/L MgCl₂ and 600 mM/L acetic acid.

To form a medical fluid, other electrolyte components may also be needed. These electrolyte components are typically dosed in higher amounts than SSR-components. Further, their concentrations can be readily measured with a desired concentration sensor being a conductivity sensor when dosed at an amount to reach an intended concentration of the electrolyte component in the final fluid. An example electrolyte component for PD that shall be dosed in higher amounts comprises 1841.0 mmol/L NaCl, 25.0 mmol/L CaCl₂, 5.0 mmol/L MgCl₂ and 801.0 mmol/L NaLact. An example electrolyte component for EC treatment that shall be dosed in higher amounts is SelectCart^{™} comprising sodium chloride (NaCl). These example electrolyte components are not considered to be SSR-components, instead, they are regarded as regular electrolyte components.

An SSR-component and/or an electrolyte component is typically embodied as liquid concentrates. The liquid concentrate may have been prepared at a manufacturer, or been prepared from a dry concentrate, e.g., a powder, granules or tablets, or more concentrated liquid concentrate, and pure water, at the site of the mixing system. A liquid concentrate is typically contained in a container, e.g., a bag. A container may contain concentrate for one or several treatments. The container is typically disposable.

The resulting conductivity of a liquid concentrate diluted with water will reflect the contribution to conductivity from all individual electrolytes in the liquid concentrate. In this disclosure, the electrolytes forming a liquid concentrate, are collectively referred to as an electrolyte component. A liquid concentrate, including electrolytes and/or non-electrolytes such that the contribution from all electrolytes and/or non-electrolytes in the liquid concentrate has a small conductivity response such as an SSR-component, is referred to as an SSR-component.

As explained, the medical fluid is for example a liquid to be used by the apparatus itself. Such medical fluid may include glucose from a glucose concentrate. The medical fluid may alternatively be an IV fluid, for example, a nutrient solution. A nutrient solution may also include glucose from a glucose concentrate. In such cases, and the like, the glucose concentrate may be regarded as an SSR-component which is mixed with pure water at the point of care to provide the medical fluid.

A medical fluid such as a treatment fluid for PD comprises electrolytes including a buffer, and an osmotic agent. The electrolytes include sodium, calcium, magnesium and optionally potassium. The buffer may be lactate, and the osmotic agent glucose and/or Icodextrin. In systems where the medical fluid for PD is mixed at the point of care, the components of the medical fluid may be provided as one concentrate comprising the osmotic agent and an additional concentrate comprising the electrolytes and the buffer. Thereby formation of glucose degradation products (GDP's) is minimized. Also, having the osmotic agent in a separate container enables individualized dosing of the same agent. The osmotic agent concentrate may in such a system be regarded as an SSR-component. The electrolytes and buffer concentrates may be regarded as an electrolyte component. However, in other examples, the division is different, for example for producing other treatments fluids for PD such as Dianeal^{®} or Physioneal^{®}. For example, for Physioneal^{®}, glucose, CaCl₂, MgCl₂ and acid may be contained in one concentrate container (then regarded as the SSR-component) and NaCl and NaHCO₃ may be contained in another, separate container (regarded as an electrolyte component). A final mixed medical fluid for PD may be composed of glucose (C₆H₁₂O₆), sodium (Na⁺), calcium (Ca²⁺), magnesium (Mg²⁺), chloride (Cl⁻), and lactate (Lact). In one example embodiment for PD, the final predetermined composition or concentration in the dialysis fluid for PD comprises 75.5 mmol/L (1.36%) glucose, 132 mmol/L Na⁺, 1.25 mmol/L Ca²⁺, 0.25 mmol/L Mg²⁺, 95.05 mmol/L Cl⁻, and 40 mmol/L lactate. However, the glucose concentration may vary from 1-5%, for example 1.36%, 2.27%, 3.86 or 4.25%.

A medical fluid such as a treatment fluid for EC treatment comprises pure water, an acid component and a base component. The acid component typically comprises sodium (Na⁺), potassium (K⁺), calcium (Ca²⁺), magnesium (Mg²⁺), glucose and either of, or a combination of, acetic, lactic or citric acid. The respective salt of the acids may also be in the component. The acid component may also include phosphate. The base component normally contains only sodium bicarbonate (NaHCO₃) but may in some embodiments also include sodium chloride (NaCl) (if not included in the acid component). The acid component concentrate and the bicarbonate component concentrate are kept in separate containers before mixing. One example of a base component is BiCart^{™} which contains dry sodium bicarbonate (NaHCO₃) powder. It is dissolved with pure water "online" before being mixed with the rest of the components of the produced dialysis fluid. Constituents of the acid component, e.g., potassium, may be contained as a concentrate in a separate container to enable individual dosage of the same. Such a separate concentrate, giving a minor contribution to the final conductivity, is here considered to be an SSR-component. The acid component (now without, e.g., the potassium) is one electrolyte component, and the base component is an additional electrolyte component. A medical fluid for EC treatment may e.g., comprise glucose (C₆H₁₂O₆), sodium (Na⁺), calcium (Ca²⁺), magnesium (Mg²⁺), chloride (Cl⁻), bicarbonate (HCO₃⁻), potassium (K⁺) and citrate (C₆H₅O₇³⁻). For EC treatment, in one example embodiment, the final predetermined composition or concentration in a medical fluid for HD comprises 1 g/L glucose, 140 mmol/L Na⁺, 1.50 mmol/L Ca²⁺, 0.5 mmol/L Mg²⁺, 109 mmol/L Cl⁻, 34 mmol/L HCO₃⁻, 2 mmol/L K⁺, and 1 mmol/L C₆H₅O₇³⁻.

Pure water here means water that has been purified to a desired purity level, for example water for dialysis, ultrapure water, or water for injection. Water for dialysis is defined according to ISO/ANSI/AAMI 23500-3:2019, defining maximum concentrations for a range of substances, and requires Total viable microbial counts < 100 CFU/ml and Endotoxins < 0.25 EU/ml. Ultrapure water may be defined as Type I water per ASTM and ISO Water Quality Standards for Laboratory-Grade Water, and quality requirements are documented by ASTM D5127 "Standard Guide for Ultra-Pure Water Used in the Electronics and Semiconductor Industries" and SEMI F63 "Guide for ultrapure water used in semiconductor processing". Water for injection is for example defined according to USP 39 NF and USP 643 NF, requiring conductivity < 1.3 uS/cm at 25°C, Total Organic Carbon < 500 ppb, Bacteria < 10 CFU/100 ml and Endotoxins < 0.25 IU/ml.

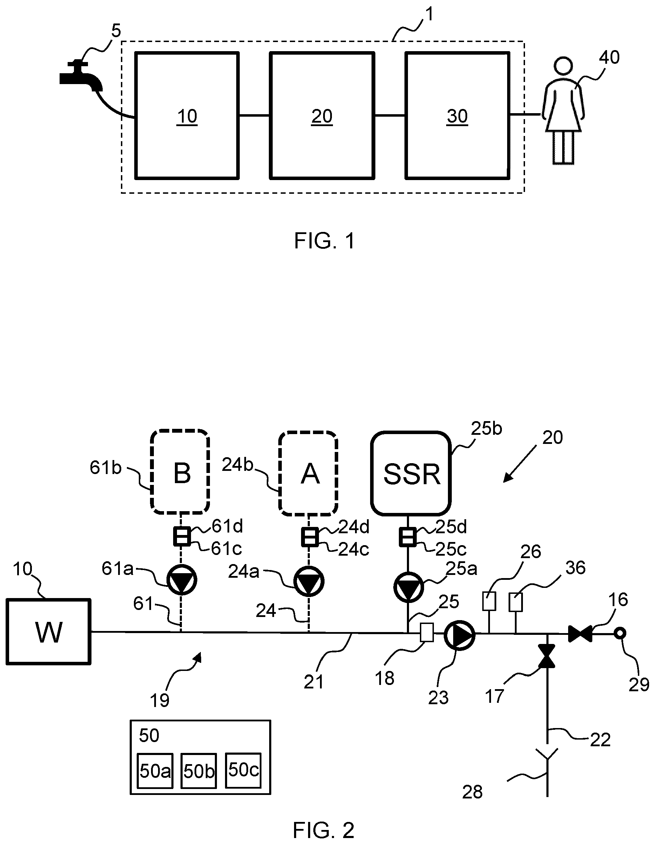

Fig. 1 is a schematic view of an example dialysis treatment system 1. The dialysis treatment system 1 is here exemplified with a water purification system 10, a mixing system 20 and a treatment delivering system 30. Any of these systems 10, 20, 30 may be integrated units or stand-alone units. For example, the water purification system 10 and the mixing system 20 may be integrated into one unit that is, for example, not easily separable.

The water purification system 10 treats one or more of water from tap 5, used medical fluid and/or waste fluid, and/or extracts water from air, and delivers pure water of a desired purification degree to the mixing system 20. The water purification system 10 may include one or more of sediment filters, carbon beds, reverse osmosis technology, forward osmosis technology, membrane distillation technology, nanofilters, ultrafilters, ion exchange technology, ultraviolet light, heaters or sterile filters. The water purification system 10 is connected to a water source, here a tap 5.

The mixing system 20 receives pure water from the water purification system 10. The mixing system 20 mixes the pure water with concentrates to provide a medical fluid to the treatment delivering system 30. The concentrates are provided in concentrate containers that are connected to the mixing system 20.

The treatment delivering system 30 receives the medical fluid from the mixing system 20. The treatment delivering system 30 is, or includes, for example a cycler or a dialysis machine. In case of a cycler, a catheter connects the treatment delivering system 30 to the patient's 40 abdomen. In case of a dialysis machine, the medical fluid is directed to a dialyzer where the fluid is passed on one side of the membrane of the dialyzer, and blood from the patient 40 is passed on the other side of the membrane. The medical fluid may also be used as replacement fluid/infusion fluid and infused into the blood upstream or downstream the dialyzer. The medical fluid may also be collected in a medical fluid bag or container (not shown) in the treatment delivering system 30 before it is further transported to the patient or dialysis system (dialyzer or upstream/downstream dialyzer). The treatment delivering system 30 is among several tasks responsible for safely delivering treatment to the patient, which includes handling of the medical fluid and to remove waste fluid from the patient.

Hereafter several different examples of mixing systems 20 will be described, that can be used in the system 1 of Fig. 1. The mixing systems 20 described herein are arranged for producing the fluids, including the mixing, at a point of care, for example in a home or in an Intensive Care (IC) unit. Hence, the mixing systems 20 as described herein may be configured to produce one or more medical fluids, for PD or EC treatment, to be used in any of PD treatment, HD, HF, HDF treatment, IC treatment, CRRT, IHD, SLED/SLEDD, or EDD, or in the mixing system 20 itself. In particular, the medical fluid may be any of a fluid to be used in the mixing system 20, an IV fluid, a dialysis fluid, an infusion fluid, a replacement fluid, or a substitution fluid. Each of the exemplified mixing systems 20 herein is arranged for so called in-line mixing of components of a medical fluid having a final predetermined composition of pure water, a small-signal-response, SSR-component and optionally at least one electrolyte component. In this disclosure, in-line mixing refers to mixing the components of the medical fluid in a line, e.g., a main fluid line 21 (see Figs. 2, 7, 10), that may include one or more fluid lines, of the mixing system 20 while the components are added as concentrates from containers to the main fluid line 21. The main fluid line 21 may be provided with mixing means for making sure that the produced fluid is homogenous when reaching an end point of the system. Examples of such mixing means is one or more small mixing chambers, static mixers, or enlargements in main fluid line that support mixing of the fluid. However, the fluid to be mixed is continuously flowing in the main fluid line 21, and then also through the mixing chamber(s)/static mixer(s)/enlargement(s). Such mixing may also be referred to as "online" mixing.

Fig. 2 is a schematic view of an example mixing system 20. This example mixing system 20 illustrates several general features of all the mixing systems as illustrated herein, and the same references in the figures refer to the same features throughout the disclosure.

The mixing system 20 comprises a fluid path 19. The fluid path 19 is in one embodiment a durable path. The fluid path 19 is typically incorporated in a housing (not shown) with ports for inlet(s)/outlet(s) to the fluid path 19. In another embodiment, the fluid path 19 is disposable. The fluid path 19 is then attached to the mixing system 20 by a user before mixing starts. The fluid path 19 includes one or more fluid lines and other components. In this disclosure, a line may be a pipe, a tube and/or a hose. The fluid path 19 comprises a main fluid line 21. The main fluid line 21 includes one, or several interconnected, fluid lines. The main fluid line 21 is arranged to be connected to a source of pure water 10. The mixing system 20 further comprises a main pump 23. The main pump 23 is arranged to the main fluid line 21 to provide a main flow rate *Qₘ* ml/min in the main fluid line 21. In one embodiment, the main pump 23 is a volumetric pump, for example a piston pump. In other embodiments, the main pump 23 is a non-volumetric pump and uses flow sensing feedback for its control. The mixing system 20 further comprises a concentration sensor 26 configured to sense a concentration of the fluid in the main fluid line 21, here positioned downstream the main pump 23. The fluid path 19 further comprises an SSR-component line 25. The SSR-component line 25 is, at one end, fluidly connected to the main fluid line 21 upstream the main pump 23 and the concentration sensor 26. At another end, the SSR-component line 25 is provided with an SSR-component line connector 25c configured to be connected to a mating SSR-component container connector 25d of an SSR-component container 25b. Hence, the SSR-component line 25 is fluidly connected to the main fluid line 21 and provided with an SSR-component line connector 25c configured to be connected to an SSR-component container 25b. In some embodiments, the fluid path 19 comprises one or more electrolyte component lines 24, 61, as indicated with dashed lines. Each electrolyte component line 24, 61 is, at one end, connected to the main fluid line 21, and, at another end, provided with an electrolyte component connector 24c, 61c configured to be connected to a mating electrolyte component container connector 24d, 61d of an electrolyte component container 24b, 61b. Hence, in some embodiments, one or more electrolyte component lines 24, 61 are fluidly connected to the main fluid line 21 and provided with a respective electrolyte component connector 24c, 61c configured to be connected to an electrolyte component container 24b, 61b. In other words, in some embodiments, an electrolyte component line 24 is fluidly connected to the main fluid line 21 and provided with an electrolyte component connector 24c configured to be connected to an electrolyte component container 24b. In other embodiments, an additional electrolyte component line 61 is also fluidly connected to the main fluid line 21 and provided with an additional electrolyte component connector 61c configured to be connected to an additional electrolyte component container 61b.

The mixing system 20 further comprises an SSR dosing mechanism 25a. The SSR dosing mechanism 25a is arranged to the SSR-component line 25 to provide a dosing rate of the SSR-component in the SSR-component line 25. More in detail, the SSR dosing mechanism 25a is arranged to provide the SSR-component at a dosing rate into the main fluid line 21 upstream the concentration sensor 26. In one embodiment, the SSR mechanism 25a is a volumetric pump, for example a piston pump. In another embodiment, the SSR mechanism 25a is a non-volumetric pump and uses flow sensing feedback for its control. In still another embodiment, the SSR mechanism 25a includes one or more valves. In some embodiments, the mixing system 20 further comprises one or more electrolyte dosing mechanisms 24a, 61a. Each such electrolyte dosing mechanism 24a, 61a is then arranged to a respective electrolyte component line 24, 61 to provide a dosing rate of the electrolyte component in the electrolyte component line 24, 61. More in detail, each such electrolyte dosing mechanism 24a, 61a is arranged to provide one of the at least one electrolyte components at a dosing rate into the main fluid line 21 upstream the concentration sensor 26. In other words, in some embodiments, an electrolyte dosing mechanism 24a is arranged to the electrolyte component line 24 to provide an electrolyte component at a dosing rate into the main fluid line 21 upstream the concentration sensor 26, 60. In some embodiments, an additional electrolyte dosing mechanism 61a is arranged to the additional electrolyte component line 61 to provide an additional electrolyte component at a dosing rate into the main fluid line 21 upstream the concentration sensor 26. In one embodiment, an electrolyte dosing mechanism 24a, 61a is a volumetric pump, for example a piston pump. In another embodiment, an electrolyte dosing mechanism 24a, 61a is a non-volumetric pump and uses flow sensing feedback for its control. In another embodiment, an electrolyte dosing mechanism 24a, 61a includes one or more valves.

The concentration sensor 26 is arranged to the main fluid line 21 downstream the main pump 23. Alternatively, the concentration sensor 26 is arranged upstream the main pump 23 but downstream any concentrate and pure water addition locations in the main fluid line 21. The concentration sensor 26 is arranged to sense concentration of the fluid in the main fluid line 21 downstream the main pump 23 (or upstream the main pump 23 if placed upstream the same pump). The fluid in the main fluid line 21 may be pure water, a mixture of pure water and the SSR-component, or a mixture of pure water, the SSR-component and the one or more electrolyte components, depending on which stage the mixing process is in and the type of medical fluid. Especially, the concentration sensor 26 is configured to sense concentration of the final medical fluid. In some embodiments, the final medical fluid is the medical fluid that is to be delivered to the treatment delivering system 30. The mixing system 20 may also be provided with another concentration sensor 36 that is arranged to measure concentration on the same fluid as the concentration sensor 26. The concentration sensor 26 is used for control of the mixing system 20. The other concentration sensor 36 is used for protective measurement. In case their measurements differ, there is a malfunction in the concentration measurement(s). A concentration sensor reading may be temperature compensated using a temperature measurement of a temperature sensor. Hence, a concentration sensor as disclosed herein may also include a temperature sensor (not shown) configured to sense temperature of the same fluid as the concentration sensor is sensing. The concentration sensor is for example a conductivity sensor or a glucose sensor. Such conductivity sensor may also be referred to as a conductivity cell. A concentration sensor is typically configured to measure in a certain measuring range with a predefined accuracy. The measuring range is the range of measured values for the concentration, in which defined, agreed, or guaranteed error limits are not exceeded.

The main fluid line 21 is configured to be connected at one end point 29 to the treatment delivering system 30, for delivery of produced medical fluid to the treatment delivering system 30. In one embodiment, the main fluid line 21 is provided with a connector at the end point 29, wherein the connector is configured to be connected to a mating connector (not shown) of the treatment delivering system 30. The fluid path 19 further comprises a waste line 22 fluidly connected to the main fluid line 22. The waste line 22 in Fig. 2 is fluidly connected to the main fluid line 22 downstream the main pump 23 and the concentration sensors 26, 36, and upstream the end point 29. The waste line 22 is configured to transport fluid discarded for use to a drain 28. Such fluid is for example fluid that does not fulfill requirements, e.g., on concentration, for a final medical fluid. In some embodiments, a first valve 16 is arranged to control the flow of fluid in the main fluid line 21 downstream the diversion to the waste line 22. In some embodiments, a second valve 17 is arranged to control the flow of fluid in the waste line 22. The first valve 16 and second valve 17 may be exchanged for a three-way valve (not shown) fluidly arranged to the main fluid line 21 and the waste line 22. Until the medical fluid is mixed properly and finally for treatment, a control arrangement 50 causes first valve 16 to be closed and second valve 17 to be open so that medical fluid under preparation is delivered through second valve 17 and waste line 22 to drain 28. Once the medical fluid is mixed properly and ready for treatment, control arrangement 50 causes first valve 16 to open and second valve 17 to close so that ready medical fluid flows through end point 29. In other words, in some embodiments, the mixing system 20 comprises one or more valves 16, 17 configured to be controlled by the control arrangement 50 to direct fluid in the main fluid line 21 to waste 28 or to an end point 29 of the main fluid line 21.

The fluid path 19 may also include at least one mixing chamber 18. One such mixing chamber 18 is arranged to the main fluid line 21 upstream the main pump 23 and downstream any concentrate connection point and pure water inlet point to the main fluid line 21. A mixing chamber 18 has an inlet where fluid is received from the main fluid line 21 into an inner compartment and an outlet where the fluid is outputted from the inner compartment to the main fluid line 21. A purpose of the mixing chamber 18 is to promote mixing of the fluid, and, in some embodiments, to remove gas from the fluid. During use, fluid is continuously flowing through the mixing chamber 18. It can typically accommodate a volume of 10-200 ml of fluid.

The source of pure water is for example the water purification system 10 in Fig. 1. In one embodiment, the water purification system 10 and the mixing system 20 share the same fluid path 19. The main fluid line 21 may then continue into and within the water purification system 10. In another embodiment, the main fluid line 21 is provided with a pure water inlet connector (not shown) for connecting to the source of pure water. The water purification system 10 may then include a mating pure water outlet connector (not shown) provided in its fluid path. By connecting the pure water outlet connector to the pure water inlet connector, the two systems 10, 20 becomes fluidly connected. In operation, pure water with a desired purification degree flows into the main fluid line 21, the flow for example provided with a pumping mechanism inside the water purification system 10 or by the main pump 23. The speed of the main pump 23 determines the flow rate in the main fluid line 21. If only pure water is flowing in the main fluid line 21, the flow rate of pure water outputted or pulled from the water purification system 10 will be equal to the main flow rate pumped with the main pump 23.

The mixing system 20 further comprises a control arrangement 50 configured to control operation of the mixing system 20. In some embodiments, the control arrangement 50 is also configured to control operation of the water purification system 10. The control arrangement 50 may be configured to be controlled by another control arrangement (not shown) arranged e.g., in the treatment delivering system 30. The control arrangement 50 includes processing means 50a and memory means 50b. The processing means 50a may include one or more processors. The memory means 50b may include one or more memories. The control arrangement 50 further includes interface means 50c. The interface means 50c may include one or more of a data interfaces for transmitting and receiving data and/or signals, a user interface for communicating information such as operation data and alarms, as well as receive user input to the mixing system 20. The control arrangement 50 is configured to receive sensed data from the one or more concentration sensors 26, (from concentration sensor 36 for protective use), operation data from the pump 23 and mechanisms 24a, 25a, 61a, etc. The control arrangement 50 is further configured to send control data and/or signals to the pump 23, mechanisms 24a, 25a, 61a, and to other components such as valves 16, 17, etc. The communication may be wired or wireless. A user may start and/or stop a medical fluid mixing process by giving input to the mixing system 20 via the user interface. A user may also follow the progress of the production of medical fluid, via the user interface.

In more detail, the control arrangement 50 is configured to provide, using the main pump 23, a flow of fluid comprising pure water from the source of pure water in the main fluid line 21. To accomplish this, the control arrangement 50 sends a control signal to the main pump 23 to provide a desired flow rate in the main fluid line 21. As a response, the main pump 23 starts pumping with the desired flow rate, and thereby pulls water from the source of water with the desired flow rate. In some embodiments, the control arrangement 50 is configured to provide a mixture of pure water from the source of pure water and an electrolyte component from the electrolyte component container 24b in the main fluid line 21. To accomplish this, the control arrangement 50 additionally sends a control signal to the electrolyte component mechanism 24a to provide a desired flow rate of the electrolyte component from the electrolyte component container 24b. As a response, the electrolyte component mechanism 24a starts pumping with the desired flow rate of the electrolyte component, which thereby flows via the electrolyte component line 24 into the main fluid line 21, where it mixes with the pure water. In some embodiments, the control arrangement 50 is configured to provide a mixture of pure water from the source of pure water, an electrolyte component from the electrolyte component container 24b and an additional electrolyte component from the additional electrolyte component container 61b in the main fluid line 21. To accomplish this, the control arrangement 50 additionally sends a control signal to the additional electrolyte component mechanism 61a to provide a desired flow rate of the additional electrolyte component from the additional electrolyte component container 61b. As a response, the additional electrolyte component mechanism 61a starts pumping with the desired flow rate of the additional electrolyte component, which thereby flows via the additional electrolyte component line 61 into the main fluid line 21, where it mixes with the pure water and the electrolyte component. The control arrangement 50 is also configured to monitor, using the concentration sensor 26, a concentration of the fluid in the fluid line 21. To accomplish this, the concentration sensor 26 sends sensed data from the concentration sensor 26 to the control arrangement 50, or the control arrangement 50 pulls the sensed data from the concentration sensor 26. The control arrangement 50 is further configured to control, using the SSR dosing mechanism 25a, the dosing rate of the SSR-component to an initial dosing rate at which the concentration monitored with the concentration sensor 26 indicates an initial concentration *c_{SSR_init}* of the SSR-component in the fluid that is greater than an intended final predetermined concentration *c_{SSR_final}* of the SSR-component in the medical fluid, in order to determine a relationship between the dosing rate of the SSR-component and the resulting concentration of the SSR-component in the fluid. To accomplish this, the control arrangement 50 sends a control signal to the SSR-component mechanism 25a to provide the initial dosing rate of the SSR-component from the SSR-component container 25b. The initial dosing rate is either predetermined or is a dosing rate where a certain concentration is achieved, as monitored with the concentration sensor 26. Hence, in some embodiment, the control arrangement 50 controls the SSR-component mechanism 25a to an initial dosing rate using feedback from the concentration sensor 26. As a response, the SSR-component mechanism 25a starts pumping with the initial dosing rate of the SSR-component, which thereby flows via the SSR-component line 25 into the main fluid line 21, where it mixes with the pure water. In some embodiments, it also mixes with an electrolyte component, and in still further embodiments, it also mixes with an additional electrolyte component, if provided in the main fluid line 21. The control arrangement 50 is further configured to downscale, using the SSR dosing mechanism 25a, the dosing rate of the SSR-component to a final dosing rate *Q_{SSR_final}* at which the final predetermined concentration *C_{sgg f}i_{ng};* of the SSR-component in the fluid is achieved, based on the determined relationship. In order to accomplish this, the control arrangement 50 determines a final dosing rate based on the relationship and sends a control signal to the SSR dosing mechanism 25a with the final dosing rate. As a response, the SSR-component mechanism 25a starts pumping with the final dosing rate.

To mix a medical fluid, one or more concentrates and pure water are used. Such concentrates, also referred to as components, are schematically illustrated in Fig. 2 with the SSR-component container 25b, an electrolyte component container 24b and an additional electrolyte component container 61b. However, in some embodiments, the SSR-component is being mixed with only pure water, and therefore the electrolyte component containers 24b, 61b are denoted with dashed lines. The flow rate in the main fluid line 21 is typically set to a predetermined main flow rate as desired by the receiving consumer/device, e.g., the treatment delivering system 30 or a container (not shown). The predetermined main flow rate is for example 10-1000 ml/min, for example 10-60 ml/min for CRRT, e.g., 20, 25, 30, 35, 40, 45 ml/min, or 300-800 ml/min for Intermittent hemodialysis (HD/HF/HDF), or 100-500 ml/min for PD, e.g., 200, 250, 300, 350, 400, 450 or 500 ml/min. An infusion unit/pump may be arranged to pump infusion fluid from the container at an infusion rate to a patient. If only pure water is added to the main fluid line 21, the incoming pure water flow to the main fluid line 21 will have the same flow rate as the predetermined main flow rate. When the SSR-component is added, the pure water flow rate will decrease with same amount as the SSR-component dosage rate. The same will occur when the electrolyte component(s) is/are added, hence, the pure water flow rate will decrease with the electrolyte component flow rate(s). In other words, the pure water flow rate will be decreased with the SSR-component dosage rate, and if also added, the electrolyte component flow rate(s). Hence, the water purification system 10 may be configured to provide a varying amount of pure water to the mixing system 20. In some embodiments, the pure water flow rate is automatically adjusted when another component is introduced into the main flow line 21 such that the flow rate in the main flow line 21 remains constant. In other embodiments, the pure water flow rate is controlled to be adjusted to accommodate the other component(s) in the main flow line 21, such that the flow rate in the main flow line 21 remains constant. For example, the pure water flow rate is reduced with the other component's flow rate(s). The pure water not used by the mixing system 20 may be recirculated in the water purification system 10. Alternatively, the mixing system 20 pulls water from a pure water tank (not shown) inside the water purification system 10 or inside the mixing system 20 that is continuously or repeatedly replenished from the water purification system 10.

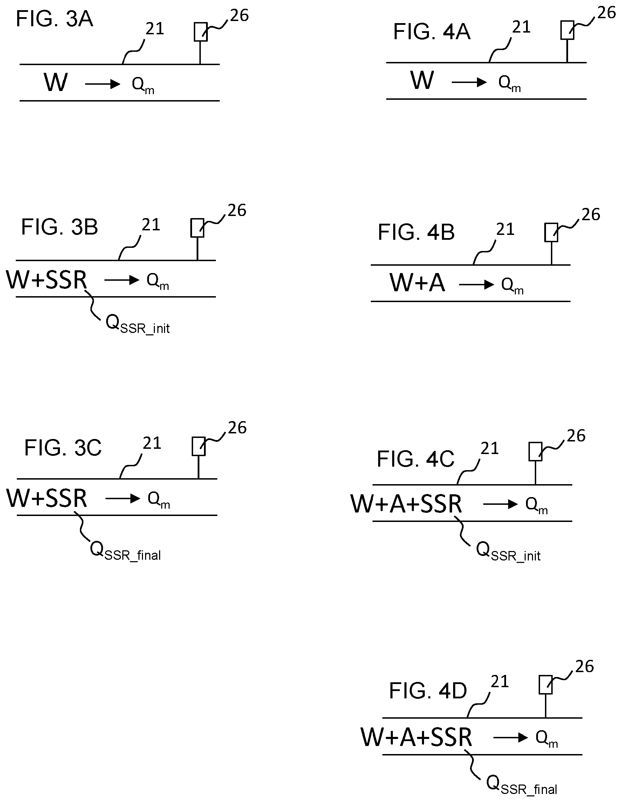

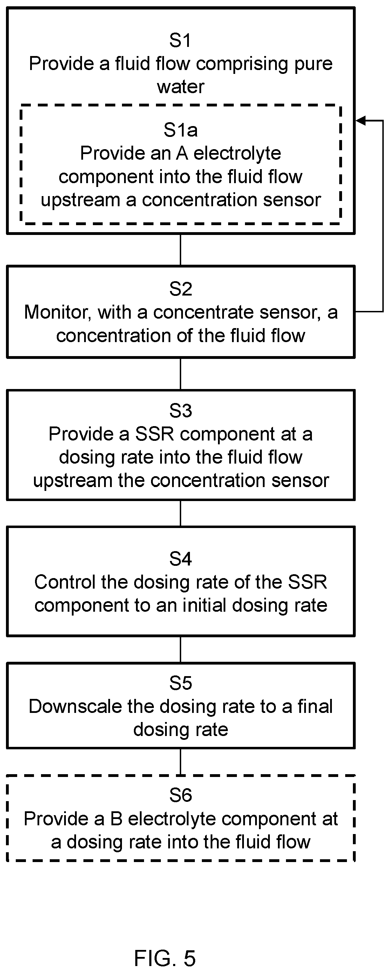

In the following, some examples of mixing including dosing of an SSR-component as illustrated in Figs. 3 and 4, and by the flow chart in Fig. 5, will be explained. The system performing the mixing may be the mixing system 20 in Fig. 2. In more detail, the flow chart in Fig. 5 illustrates method steps that can be implemented by the control arrangement 50 in Fig. 2. Hence, the control arrangement 50 is configured to perform all the steps, examples and embodiments outlined below in relation to the Figs. 3A to 6.

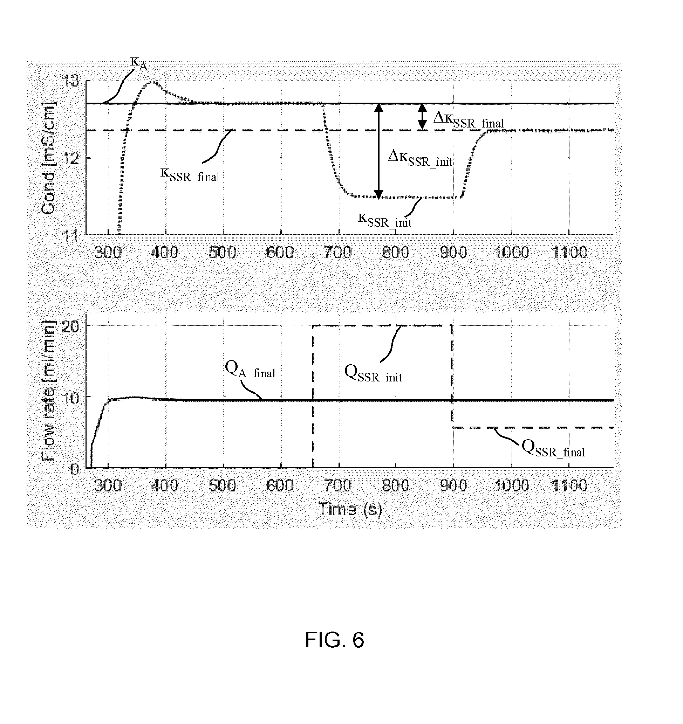

In these mixing examples, and in all other mixing examples described herein, the mixing includes temporarily overdosing the SSR-component to be able to accurately sense the concentration of the SSR-component, and subsequently downscaling the dosing rate of the SSR-component to a final dosing rate, which complies with a prescription including a final concentration of the SSR-component in the final medical fluid. Hence, what is described regarding such overdosing and downscaling in relation to the examples in Figs. 3 to 5, can be equally applied to the examples described in relation to the examples described in relation to the Figs. 6 to 11F, but is not always repeated for ease of illustration.

Figs. 3A-3C illustrate one example of dosing an SSR-component, i.e., an SSR-concentrate, using the system 20 in Fig. 2. A part of the main fluid line 21 of the mixing system 20 is schematically illustrated in the figures, together with the concentration sensor 26. In this example, the concentration sensor 26 is a conductivity sensor. Further, the SSR-component is an electrolyte component that is to be dosed in a small amount to the pure water in the main fluid line 21. Hence, when the SSR-component is added to the pure water in the main fluid line 21, the conductivity of the mixed fluid will increase compared to the conductivity of the pure water. The desired final composition of medical fluid is predetermined and known, and hence also the intended final predetermined concentration of the SSR-component in the medical fluid, *c_{SSR_final}.* However, the final concentration of the SSR-component in the medical fluid is so small that the continuously dosed flow rate to provide the final concentration of the SSR-component would not give a reliable conductivity measurement with the concentration sensor 26. Reference is also made to the flow chart in Fig. 5 for explaining the example method.

In a first step S1, a flow of fluid comprising pure water (W) is provided in the main fluid line 21, e.g., with a flow rate of *Qₘ* ml/min. The flow of pure water is provided as has been previously described. In other words, in some embodiments, the providing S1 a flow of fluid comprises controlling a main flow rate of the fluid flow to be a predetermined fluid rate of medical fluid. The main flow rate may be controlled using a main pump 23 arranged to the main fluid line 21. The predetermined fluid flow rate may be a flow rate of medical fluid configured for a downstream located device or user. In more detail, the flow of fluid comprises only pure water, hence, no other fluids or concentrates are added to the main fluid line 21 at this stage. This is illustrated in Fig. 3A with the flow of pure water W in the main fluid line 21, where the arrow indicates the direction of the flow. As only pure water is flowing in the main fluid line 21, the flow rate pumped with the main pump 23 will be equal to the pure water flow rate.

In a second step S2, the conductivity of the fluid is monitored with the concentration sensor 26. This is illustrated in Fig. 3A where the concentration sensor 26 measures the conductivity of the fluid in the main fluid line 21. In this disclosure, monitoring includes to measure the concentration of (a property, a substance or component in) the fluid continuously, continually, or repeatedly. The measured values of the concentration, in this example the conductivity, are provided to the control arrangement 50. As only pure water is flowing in the main fluid line 21, the expected conductivity is zero, or very close to zero. The monitoring S2 is typically performed with the same concentration sensor 26. Hence, in some embodiments, all monitoring of the concentration of the fluid in the main fluid line 21 used for control is made using the same concentration sensor 26.

In a third step S3, the SSR-component is provided at a dosing rate, with the SSR dosing mechanism 25a, into the flow of fluid upstream the concentration sensor 26. The pure water flow rate is thereby reduced with the SSR-component dosing rate, so the total flow rate in the main fluid line 21 is still the same, hence *Qₘ* ml/min.

The dosing rate of the SSR-component is thereafter controlled in an overdosing step S4, with the SSR dosing mechanism 25a, to an initial dosing rate *Q_{SSR_init}* ml/min. At the initial dosing rate *Q_{SSR_init}* the conductivity, monitored with the concentration sensor 26, indicates an initial concentration *c_{SSR_init}* of the SSR-component in the fluid. The initial concentration *c_{SSR_init}* is greater than an intended final predetermined concentration *^{C}_{SSR} __{f} iₙₐₗ* of the SSR-component in the medical fluid. The step S4 determines, e.g., establishes, a relationship between the dosing rate of the SSR-component and its resulting concentration of the SSR-component in the fluid. The relationship is determined based on the desired final concentration *c_{SSR_final}* of the SSR-component, the initial concentration *c_{SSR_init}* of the SSR-component, and the initial dosing rate *Q_{SSR_init}* of the SSR-component. It should here be mentioned that the desired final concentration *c_{SSR_final}* of the SSR-component is predetermined, but that only one of the initial concentration *c_{SSR_init}* of the SSR-component and the initial dosing rate *Q_{SSR_init}* of the SSR-component is determined precisely in beforehand. The one that is determined in beforehand is used as a control parameter by the control arrangement 50, and the other one is precisely determined in the overdosing step S4. The initial concentration *c_{SSR_init}* of the SSR-component may either be a predetermined precise value, or a predetermined range. Further, the initial dosing rate *Q_{SSR_init}* of the SSR-component may either be a predetermined precise value, or a predetermined range. Hence, different control strategies are here possible. The effect of step S4 on the flow rate is illustrated in Fig. 3B, where the total flow rate in the main fluid line 21 is still the same *Qₘ* ml/min, but the pure water flow rate W has been decreased to: *Qₘ* ml/min minus *Q_{SSR_init}* ml/min. At the initial dosing rate *Q_{SSR_init}* ml/min, the SSR-component contribution to the pure water is measurable with the concentration sensor 26.

In some embodiments, the initial concentration *c_{SSR_init}* of the SSR-component in the fluid is greater than the intended final predetermined concentration *c_{SSR_final}* of the SSR-component in the medical fluid, such that a ratio between a concentration response, here conductivity response, resulting from SSR-component dosing at the initial dosing rate *Q_{SSR_init}* and a concentration, here conductivity, signal noise is equal to or greater than a predetermined limit. The ratio may be expressed as a signal-to-noise ratio, $SNR = \frac{S}{N}$, where the level of the desired signal, *S*, is compared to a level of background noise, N. The predetermined limit of the ratio is then a predetermined minimum value of the *SNR.* The desired signal S is for example a measure of the concentration, here conductivity, response signal resulting from the SSR-dosing at the initial dosing rate *Q_{SSR_init},* measured with the concentration sensor 26. The measure is for example a magnitude or an average value. The noise N is a quantization of the concentration response signal noise, hence a dispersion of the signal from the concentration sensor 26 before the SSR-component is dosed. The noise *N* is for example predetermined, calculated by the control arrangement 50, as one or more or a variance of the signal from the sensor, a standard deviation of the signal from the sensor, a range of the signal from the sensor, an interquartile range of the signal from the sensor, a mean absolute difference of the signal from the sensor, a median absolute deviation of the signal from the sensor, an average deviation of the signal from the sensor, a trend over time of the signal from the sensor, or a derivative over time of the signal from the sensor. The larger the ratio, the better the accuracy of the measurement of the desired signal. The noise originates from the measurement itself, but also concentration variations caused by mixing homogeneity differences of the fluid. In some embodiments, one of the initial concentration *c_{SSR-init}* and the initial dosing rate *Q_{SSR_init}* of the SSR-component has been determined in beforehand to give a desired *SNR.* In other embodiments, the noise N is calculated by the control arrangement 50 based on the concentration response before the SSR-component is dosed. A required signal of the fluid when the SSR-component is dosed to achieve an SNR equal to or greater than the predetermined limit, may then be determined by multiplying the noise N with the predetermined limit. The overdose magnitude may then be adapted to the noise at hand and SSR-component. Hence, in some embodiments, the concentration, e.g., conductivity κ*ᵢₙᵢₜ*, at the initial dosing rate *Q_{SSR_init}* is a function of a size of the noise N.

The initial dosing rate *Q_{SSR_init}* is further greater than a final dosing rate *Q_{SSR_final}* of the SSR-component. The final dosing rate of the SSR-component is the dosing rate that will give the intended final concentration of the SSR-component in the medical fluid. In one embodiment, the initial dosing rate *Q_{SSR_init}* of the SSR-component is > 1 to 20 times larger than the final dosing rate *Q_{SSR_final}.* For example, it may be 1.5, 2, 5 or 10 times larger. In one example embodiment, the initial dosing rate *Q_{SSR_init}* ml/min is predetermined. It indicates a dosing rate where it is known, for example established by measurements and/or experiments, that the conductivity contribution of the SSR-component in the pure water flow can be accurately measured using the concentration sensor 26. The exact resulting conductivity at the predetermined initial dosing rate will be unknown but within a known interval around a known nominal value. The controlling S4 is then performed by controlling the dosing rate to the predetermined initial dosing rate value where it is known that the conductivity can be measured with the concentration sensor 26 with desired accuracy. In other words, the initial dosing rate here corresponds to a predetermined initial dosing rate *Q_{SSR_init}.* The exact conductivity at the predetermined initial dosing rate will in this example embodiment be unknown but is expected to be within a known interval. Hence, the controlling S4 may include controlling the dosing rate of the SSR-component until a predetermined initial dosing rate *Q_{SSR_init}* ml/min is achieved. The conductivity at the predetermined initial dosing rate is then determined. In another example embodiment, a target concentration, e.g., here represented by the conductivity κ*ᵢₙᵢₜ*, of the fluid, established by calculation typically based on experimentation, is instead predetermined. At this predetermined target concentration, the concentration sensor 26 can measure the conductivity contribution of the SSR-component in the fluid, e.g., in pure water. In other words, the conductivity measured with the concentration sensor 26 at the initial dosing rate *Q_{SSR_init}* corresponds to a predetermined target conductivity *Kᵢₙᵢₜ* of the fluid. Here the exact initial dosing rate *Q_{SSR_init}* will instead be unknown at first but is expected to be within a known interval. When the conductivity is at the predetermined target conductivity *Kᵢₙᵢₜ*, the initial dosing rate is determined. Typically, the determined initial dosing rate is sent to the control arrangement 50 as dosing rate data. Hence, the controlling S4 may include controlling the dosing rate of the SSR-component until a predetermined target concentration, e.g., here conductivity *Kᵢₙᵢₜ*, is achieved. At this predetermined target conductivity *Kᵢₙᵢₜ*, the dosing rate of the SSR-component is referred to as the initial dosing rate *Q_{SSR_init}.* Any of these control methods will also remove potential dosing errors caused by errors in nominal concentration values indicated by manufacturers of the SSR-component such as, e.g., water evaporation from the SSR-component concentrate container altering its initial concentration.

In some embodiments, the controlling S4 comprises determining the relationship as the final dosing rate *Q_{SSR_final}* being equal to a ratio between a final value *F_{final}* correlated with the desired final concentration *C_{SSR}__{f} inal* of the SSR-component and an initial value *Fᵢₙᵢₜᵢₐₗ* correlated with the initial concentration *c_{SSR_init}* of the SSR-component, multiplied with the initial dosing rate *Q_{SSR_init}.* The relationship may be expressed as follows: ${Q}_{SSR _ final} = \frac{{F}_{final}}{{F}_{initial}} ⋅ {Q}_{SSR _ init}$ (1) In one example embodiment, the final value *F_{final}* and the initial value *Fᵢₙᵢₜᵢₐₗ* are the intended final predetermined concentration *c_{SSR_final}* of the SSR-component and the initial concentration *c_{SSR_init}* of the SSR-component in the medical fluid, respectively, in percent or represented as difference values from a base/start concentration of the measured property before the SSR-component was added. The final value *F_{final}* is typically known in beforehand, and, depending on which control method that is used, one of the initial value *Fᵢₙᵢₜᵢₐₗ* and the initial dosing rate *Q_{SSR_init}* has been determined in beforehand by calculation or experimentation. Such calculations may include determining a molar conductivity versus ionic strength relationship for each substance (or electrolyte) in the component/solution/fluid. This relationship can then be used to calculate a conductivity contribution for each of the substances and then sum these to determine a total conductivity for the component/solution/fluid, to determine a final conductivity *κ_{SSSR_final}* and/or an initial conductivity κ*_{SSR_init}* which are respectively related with *c_{SSR_final}* and/or *c_{SSR_init}.* Experimentation may include determining a base/start conductivity of the measured property of the fluid before the SSR-component is added. The other of the initial value *Fᵢₙᵢₜᵢₐₗ* and the initial dosing rate *Q_{SSR_init}* is determined i.e., measured, at the controlling step S4. These examples are for illustrating the principle and more alternatives are conceivable. The ratio or quotient $\frac{{F}_{final}}{{F}_{initial}}$ is here a scaling factor that is used to scale down the initial dosing rate *Q_{SSR_init}* of the SSR-component to a final dosing rate *Q_{SSR_final}* where the correct addition of the SSR-component is obtained to achieve the final composition.

In a further step S5, the method includes downscaling, with the SSR dosing mechanism 25a, the dosing rate of the SSR-component to the final dosing rate *Q_{SSR_final}* at which the final predetermined concentration *c_{SSR_final}* of the SSR-component in the fluid is achieved, based on the determined relationship. Hence, a final dosing rate *Q_{SSR_final}* is determined, for example using the established values in Equation (1), and used for controlling the SSR dosing mechanism 25a. This is illustrated in Fig. 3C, where the total flow rate in the main fluid line 21 is still the same *Qₘ* ml/min, but the pure water flow rate W has been increased to: *Qₘ* ml/min minus *Q_{SSR_final}* ml/min compared to Fig. 3B. Hence, when the relationship has been determined, the final dosing rate *Q_{SSR_final}* has also been determined, which is the desired dosing rate of the SSR-component to provide an intended final medical fluid. Thereby the SSR-component can be dosed at a desired final dosing rate without needing to measure the conductivity contribution of the SSR-component at this final dosing rate. In some embodiments, the downscaling S5 comprises fixing a ratio between the final dosing rate *Q_{SSR_final}* of the SSR-component and the main flow rate. Thereby, if the main flow rate changes, the dosing rate of the SSR-component will also change accordingly to maintain the ratio and thereby the final composition of the medical fluid. The ratio may be referred to as a dilution ratio of the SSR-component.

For ease of illustration, the embodiments and examples herein are generally explained with concentration sensors being conductivity sensors. However, such conductivity sensor could be exchanged for a glucose sensor, in cases where the SSR-component is a glucose component, i.e., a glucose concentrate, and mixed initially with pure water only. Hence, in some embodiments, the concentration sensor 26 is a glucose sensor. The additional concentration sensor 36 may then be an additional glucose sensor used as a protective sensor. The glucose sensor is then used to determine the concentration of the SSR-component being a glucose concentrate, using the overdosing principle as explained herein. In some embodiments, the system 20 may additionally comprise a conductivity sensor, and a protective conductivity sensor, (not shown) both arranged to measure conductivity in the main fluid line 21 upstream the main pump 23. These conductivity sensors may be used to determine any other concentration of the fluid in the main fluid line 21, for example, the concentration of electrolyte components and/or concentration of an intermediate and/or the final fluid.

Hence, in one example embodiment, the concentration sensor 26 is a glucose sensor and the SSR-component is a glucose component. In such example embodiment, in a first step S1, a flow of fluid comprising pure water is provided in the main fluid line 21. The concentration of glucose in the fluid is monitored in a step S2 with the glucose sensor. The glucose component is provided into the flow of fluid upstream the concentration sensor 26. The glucose sensor monitors the concentration of glucose of the fluid now being a mix of glucose concentrate and pure water. An overdosing of glucose component is then performed in a step S4, by controlling, with the SSR dosing mechanism 25a, the dosing rate of the glucose component to an initial dosing rate at which the concentration monitored with the glucose sensor indicates an initial concentration *c_{SSR_init}* of the glucose component in the fluid that is greater than an intended final predetermined concentration *c_{SSR_final}* of the glucose component in the medical fluid, in order to determine a relationship between the dosing rate of the glucose component and the resulting concentration of the glucose component in the fluid. Thereafter the dosing rate of the glucose component is downscaled in a step S5, with the SSR dosing mechanism 25a, to a final dosing rate *Q_{SSR_final}* at which the final predetermined concentration *c_{SSR_final}* of the glucose component in the fluid is achieved, based on the determined relationship. Other steps of the method as outlined in connection with Figs. 1 to 3 may be equally applicable in connection with this example with a glucose sensor as concentration sensor.

Figs. 4A-4D illustrate another example of dosing an SSR-component, i.e., an SSR-concentrate, using the system 20 in Fig. 2. Also, in this example, a part of the main fluid line 21 of the mixing system 20 is schematically illustrated in the figures, together with the concentration sensor 26. In this example, the concentration sensor 26 is a conductivity sensor. Further, in this example, the SSR-component decreases the conductivity of the fluid into which it is added in the main fluid line 21. The SSR-component is for example a nonelectrolyte, e.g., glucose concentrate or Icodextrin. The desired final composition of medical fluid is previously predetermined and known, and hence also the intended final predetermined concentration of the SSR-component *c_{SSR_final}* in the medical fluid. However, the final concentration of the SSR-component *c_{SSR_final}* in the medical fluid is so small that the continuous dosed flow rate to provide the final concentration of the SSR-component would not give a reliable conductivity measurement with the concentration sensor 26. Also, as the SSR-component decreases the conductivity, it is necessary to first add an electrolyte component to the pure water that will increase the conductivity of the fluid to a higher conductivity, so it can be measured with a concentration sensor being a conductivity sensor. Thereafter, when the SSR-component is added, it will decrease the conductivity from the higher conductivity established by the added electrolyte component to a sufficiently lower value accurately measurable with the concentration sensor 26 being a conductivity sensor. The desired final composition of medical fluid is previously predetermined and known. Hence, also the intended final predetermined concentration of the SSR-component *c_{SSR_final}* in the final predetermined composition of medical fluid, and the final predetermined concentration of the electrolyte component *c_{A_final}* in the final predetermined composition of the medical fluid, are known. Reference is also made to the flow chart in Fig. 5 for explaining the example method.

In a first step S1, a flow of fluid comprising a mixture of pure water (W) and one of the at least one electrolyte components is provided in the main fluid line 21. The sum of the flows is *Qₘ* ml/min.

In a second step S2, the conductivity of the fluid is monitored by the concentration sensor 26. The first step S1 and the second step S2 are typically performed simultaneously, hence, while fluid is flowing in the main fluid line 21 the conductivity of the fluid will be monitored. Pure water is provided in the same way as in the example explained with reference to Fig. 3A to 3C and typically has the same purity. Hence, as illustrated in Fig. 4A, a flow of only pure water W is first provided in the main fluid line 21, where the arrow indicates the direction of the flow. When only pure water is flowing in the main fluid line 21, the flow rate provided with the main pump 23 will be equal to the pure water flow rate.

Compared to the example in Fig. 3A to 3C, the present example comprises also adding an electrolyte component into the main fluid line 21, before the SSR-component is added. Hence, the providing includes providing S1a the electrolyte component, with an electrolyte component dosing mechanism 24A, at a dosing rate into the flow of pure water in the main fluid line 21 to form the flow of fluid comprising a mixture of pure water and the electrolyte component upstream the concentration sensor 26. This is illustrated in Fig. 4B, where a dosing rate of the electrolyte component "A" has been added to the flow or pure water. The pure water flow rate is thereby reduced with the electrolyte component dosing rate, so the total flow rate in the main fluid line 21 is still the same, hence *Qₘ* ml/min. With the addition of the electrolyte component, the conductivity of the fluid has been increased compared to the conductivity of the flow of only pure water. In some embodiments, the providing S1a comprises, preceding the providing of the SSR-component into the flow of fluid, controlling the dosing rate of the electrolyte component, with the electrolyte component dosing mechanism 24A, to a final dosing rate *Q_{A_final}* providing a final predetermined concentration *c_{A_final}* of the electrolyte component in the final medical fluid, based on the conductivity monitored by the concentration sensor 26 of the mixture of pure water and the electrolyte component. In other words, the electrolyte dosing mechanism 24A is controlled, with conductivity feedback from the concentration sensor 26, to provide the electrolyte component at a flow rate that gives the desired concentration of the electrolyte component in the medical fluid. A target conductivity is predetermined to give the predetermined concentration of electrolyte component for the desired final medical fluid. Hence, by controlling the dosing rate of the electrolyte component dosing mechanism 24A to a dosing rate where the conductivity of the concentration sensor 26 is at the target conductivity, the desired concentration of the electrolyte component in the medical fluid is achieved. This dosing rate is referred to as the final dosing rate *Q_{A_final}*. In some embodiments, the providing S1 comprises fixing a ratio between the final dosing rate *Q_{A_final}* of the electrolyte component and the main flow rate *Qₘ*. Thereby, if the main flow rate changes, the dosing rate of the electrolyte component will also change accordingly to maintain the ratio and thereby the final composition of the medical fluid. The total flow rate in the main fluid line 21 is still the same *Qₘ* ml/min, but the pure water flow rate W has been decreased to: *Qₘ* ml/min minus *Q_{A_final}* ml/min.

The steps S3 to S5 are thereafter performed in the same way as has been described with reference to Figs. 3A to 3C, and 5, and reference is made to these sections for explaining the method, however briefly described below.

Hence, in a third step S3, the SSR-component is provided at a dosing rate, with the SSR dosing mechanism 25a, into the flow of fluid upstream the concentration sensor 26. The fluid flow is thereby reduced with the SSR-component dosing rate, so the total flow rate in the main fluid line 21 is still the same, hence *Qₘ* ml/min.

The dosing rate of the SSR-component is thereafter controlled S4, with the SSR dosing mechanism 25a, to an initial dosing rate *Q_{SSR_init}* ml/min at which the conductivity monitored with the concentration sensor 26 indicates an initial concentration *c_{SSR_init}* of the SSR-component in the fluid. The initial concentration *c_{SSR_init}* of the SSR-component is greater than an intended final predetermined concentration *c_{SSR_final}* of the SSR-component in the medical fluid, in order to determine a relationship between the dosing rate of the SSR-component and the resulting concentration of the SSR-component in the fluid. This is illustrated in Fig. 4C. As also an electrolyte component has been added, the pure water flow rate W is decreased to: *Qₘ* ml/min minus *Q_{SSR_init}* minus *Q_{A_final}* ml/min after step S4 as illustrated in Fig. 4C. For the signal-to-noise ratio, $SNR = \frac{S}{N}$, the signal S is for example the measured conductivity response at the initial dosing rate *Q_{LCR_init},* and the noise quantification N is for example the standard deviation of the measured conductivity response at the final dosing rate *Q_{A_final}* measured during a time period. The noise quantification may be determined beforehand for a given system or determined by the control arrangement 50 while performing the method, and based on conductivity measurements during the time period.

In the further step S5, the method includes downscaling, with the SSR dosing mechanism 25a, the dosing rate of the SSR-component to the final dosing rate *Q_{SSR_final}* at which the final predetermined concentration *c_{SSR_final}* of the SSR-component in the fluid is achieved, based on the determined relationship. The pure water flow rate W is then decreased to: *Qₘ* ml/min minus *Q_{SSR_final}* minus *Q_{A_final}* ml/min after step S5 as illustrated in Fig. 4D. Also, the conductivity at the initial dosing rate *Q_{SSR_init}* corresponds to a predetermined target conductivity *Kᵢₙᵢₜ* of the fluid determined also based on both the added conductivity from the electrolyte component and the SSR-component. In some embodiments, the intended final predetermined concentration *c_{SSR_final}* of the SSR-component in the medical fluid is in a range up to 5 %, e.g., 1.36%., 2.27%, or 3.86%. An example SSR-component that has such intended final predetermined concentration is glucose in a medical fluid for PD. The predetermined initial concentration *c_{SSR_init}* of the SSR-component in the fluid may in this case then be 4 to 20 percent, more preferably 4 to 10 percent. For example, when using Equation (1) here, in one embodiment the final value *F_{final}* is 1.36% and the initial value *Fᵢₙᵢₜᵢₐₗ* is 5%. In another example embodiment, the final value *F_{final}* and the initial value *Fᵢₙᵢₜᵢₐₗ* are the intended final predetermined concentration *c_{SSR_final}* of the SSR-component in the medical fluid and the predetermined initial concentration *c_{SSR_init}* of the SSR-component, respectively, established as conductivity differences in the fluid as measured from a common baseline. The common baseline may be established by experimentation or calculations.

In some embodiments, the method comprises providing S6 an additional electrolyte component, with an additional electrolyte component dosing mechanism 61A, at a dosing rate into the flow of fluid in the main fluid line 21 to form a mixture of pure water, the electrolyte component, the SSR-component and the additional electrolyte component. Thereby, a medical fluid can be made using three separate concentrates and pure water. In some embodiments, the providing S6 comprises controlling the dosing rate of the additional electrolyte component, with the additional electrolyte component dosing mechanism 61A, to a final dosing rate *Q_{B_final}* providing a final predetermined concentration *c_{B_final}* of the electrolyte component in the final medical fluid, based on the conductivity monitored by the concentration sensor 26 of the mixture of pure water, the electrolyte component and the additional electrolyte component. In other words, the additional electrolyte dosing mechanism 61A is controlled, with conductivity feedback from the concentration sensor 26, to provide an additional electrolyte component at a flow rate that gives the desired concentration of that additional electrolyte component in the medical fluid. A target conductivity is predetermined to give the predetermined concentration of the additional electrolyte component for the desired medical fluid. Hence, by controlling the dosing rate of the additional electrolyte component dosing mechanism 61A to a dosing rate where the conductivity of the concentration sensor 26 is at the target conductivity, in which the additional electrolyte component has been included, the desired concentration of the additional electrolyte component in the medical fluid is achieved. The additional electrolyte component may be referred to as the electrolyte component B.

In some embodiments, the additional electrolyte component is added already in step S1. The SSR-component will then be added to a mix of pure water, the electrolyte component and the additional electrolyte component. Hence, in such case, the providing S1 a flow of fluid comprises providing an additional electrolyte component, with an additional electrolyte component dosing mechanism 61A, at a dosing rate into the flow of pure water in the main fluid line 21 to form a flow of fluid comprising a mixture of pure water, the electrolyte component, and the additional electrolyte component upstream the concentration sensor 26. In some embodiments, the providing S1 a flow of fluid comprises, preceding the providing of the SSR-component into the flow of fluid, controlling the dosing rate of the additional electrolyte component, with the additional electrolyte component dosing mechanism 61a, to a final dosing rate *Q_{B_final}* providing a final predetermined concentration *c_{B_final}* of the additional electrolyte component in the final medical fluid, based on the concentration monitored by the concentration sensor 26 of the mixture of pure water, the electrolyte component and the additional electrolyte component. The overdosing of the SSR-component is then made in relation to the fluid comprising the mixture of pure water, the electrolyte component and the additional electrolyte component. As understood, more electrolyte components may be added before or after the SSR-component is dosed, if required for the overdosing or by the final fluid composition.

A target conductivity is typically predetermined. However, an actually achieved conductivity, i.e., the conductivity response, may to some extent differ from the target conductivity. Hence, a subsequent target conductivity that rely on a previous conductivity control step may be corrected based on the previous conductivity response. Such procedure may enhance the accuracy of the control.

Using a master conductivity cell/sensor as described herein, i.e., the concentration sensor 26, means all mixing steps are setup using the same cell/sensor.

Fig. 6 is illustrating, in two diagrams, an example dosing of an SSR-component that decreases the conductivity of the fluid to which it is added. In this example, the SSR-component comprises glucose, and the electrolyte component comprises the substances NaCl, CaCl₂, MgCl₂ and a buffer NaLact. The final medical fluid to be mixed is for example the fluid product Dianeal^{®}. In some embodiments, the SSR-component is a liquid glucose concentrate comprising between 40-75% of glucose. For example, 40, 45, 50, 55, 60, 65, 70 or 75% of glucose. As understood, the electrolytes (Na, Ca, Mg, Lact, Cl) increases the conductivity of the solution to which they are added, in this case pure water.

Alternatively, the SSR-component comprises one or more conductive substances and one or more nonconductive substances, such that the resulting SSR-component decreases the conductivity of the fluid. One such example SSR-component comprises glucose, which lowers the conductivity, and hydrochloric acid (HCl), which increases the conductivity. In total such SSR-component will decrease the conductivity of the fluid into which it is mixed.

The individual solute conductivity contributions are additive. The conductivity response from the electrolytes (the electrolyte component), in its intended dosage (nominal dosing rate) for the final medical fluid, is large and suitable for direct control. The conductivity response from the glucose (the SSR-component), in its intended dosage (nominal dosing rate) for the final medical fluid, is however small and gives and unfavorable conductivity signal-to-noise relation.

The diagrams in Fig. 6 give an overview of the electrolyte and SSR-component control steps S1 to S5 performed to find the flow rates of the dosing mechanisms that yields the correct fluid composition. In a non-limiting embodiment, the concentration sensor 26 has a measuring range of 0.1 to 50 mS/cm, more preferably of 5 to 20 mS/cm.

The upper diagram in Fig. 6 illustrates the conductivity in mS/cm during dosing of the electrolyte component and the SSR-component. The solid line represents a first target conductivity κ*_{A}* for the mixture of the electrolyte component and pure water. The first target conductivity κ*_{A}* is calculated based on the desired final electrolyte component composition and conductivity equations for each solute/electrolyte present in the mixture. As should be understood, the electrolyte component may include different kinds of dissolved substances, and the first target conductivity corresponds to the sum of conductivities from all these dissolved substances. The first target conductivity κ*_{A}* corresponds to a final predetermined concentration of the electrolyte component in the final predetermined composition of the medical fluid. In one example embodiment, the first target conductivity κ*_{A}* is 12.686 mS/cm. The dashed line in the upper diagram represents a medical fluid target conductivity κ*_{SSR_final}* of the mixture of the SSR-component, the electrolyte component and pure water. The medical fluid target conductivity κ*_{SSR_final}* is calculated based on the final predetermined composition of the medical fluid comprising purified water, the electrolyte component and the SSR-component. The medical fluid target conductivity *K_{SSR_final}* is here typically 0.35 - 1.05mS/cm lower than the first target conductivity κ*_{A}*, depending on the desired target SSR-component concentration. Hence, the SSR-component will lower the conductivity of the fluid to which it is added with 0.35 - 1.05mS/cm. This difference between the first target conductivity κ*_{A}* and the medical fluid target conductivity κ*_{SSR_final}* is hereafter denoted Δκ_{*SSR*_*final*}. The first target conductivity κ*_{A}* may not be reached exactly. The medical fluid target conductivity κ*_{SSR_final}* may here therefore be adjusted with the difference between the calculated first target conductivity κ*_{A}* and the reached first target conductivity. The dotted line represents the conductivity response as measured with the concentration sensor 26.

The lower diagram in Fig. 6 illustrates the dosing mechanisms' flow rates in ml/min. In case of a volumetric pump, the flow rate is proportional to the pump speed. The solid line represents the flow rate of the electrolyte component dosing mechanism 24a. The dashed line represents the flow rate of the SSR-component dosing mechanism 25a.

The diagrams in Fig. 6 include a first phase and a second phase. The first phase includes the electrolyte composition dosing control including steps S1 and S2 and here extends during approximately the time 250s to 650s. During the first phase, the electrolyte dosing rate is controlled, hence adjusted, as illustrated in the lower diagram, with the electrolyte dosing mechanism 24a, with conductivity feedback from concentration sensor 26 until the conductivity is sufficiently close to the first target conductivity κ*_{A}* for the electrolyte component + pure water mixture. In one embodiment, a PID controller is used to control the electrolyte component dosing mechanism 24a with conductivity feedback until certain conductivity magnitude and stability criteria are met. This adjustment is ongoing during the first phase from time 250s to approximately 500s. The electrolyte component dosing mechanism 24a flow rate *Q_{A_final}* or speed is then noted and saved in memory. Also, the electrolyte component dosing mechanism 24a to main pump 23 speed ratio is locked and stored in memory 50b. As can be seen in the lower diagram, the electrolyte dosing mechanism 24a is controlled to a flow rate of about 10 ml/min where the resulting conductivity goes beyond the first target conductivity κ*_{A}*, whereafter the flow rate decreases to just below 10 ml/min when the resulting conductivity stabilizes at the first target conductivity κ*_{A}*. Then, in the first phase, follows an optional stabilization period with locked pump speed/dosing mechanism ratio in which the conductivity is allowed to stabilize without actively controlling the electrolyte component dosing mechanism 24a using conductivity feedback. This stabilization phase lasts for about 100-200s. A filtered mean value of the resulting conductivity is thereafter saved for later use as a baseline for the second phase. In some embodiments, this stabilization period is not performed.

The second phase includes glucose composition control, hence, a kind of SSR-component dosing control. This includes providing and overdosing glucose as in step S3-S4 using the SSR dosing mechanism 25a, followed by downscaling, as in step S5, the flow rate of the SSR dosing mechanism 25a based on the Equation (1). The second phase lasts from approximately the time 650s to about 1100s. As can be seen in the lower diagram, the control includes to increase the flow rate of the SSR dosing mechanism 25a in a step from zero to 20 ml/min. Hence, the flow rate of the SSR dosing mechanism is increased to a certain target flow rate, i.e., an initial dosing rate *Q_{SSR_init}.* This target flow rate is for example a percentage of the main flow rate, for example 10 percent. As a response to the overdosing, the resulting conductivity of the fluid is reduced from the first target conductivity κ*_{A}* to a conductivity below the medical fluid target conductivity κ_{*SSR*_*final*}. The resulting conductivity at the overdosed flow rate of the SSR dosing mechanism 25a is measured and referred to as the initial conductivity κ*_{SSR_init}*. It is here measured to be 11.5 mS/cm, and is typically in the range 10.3-11.70 mS/cm. Here, the magnitude of the overdosing of the glucose component is chosen to cause a glucose concentration of between 4 to 10% in the mixed fluid (nominal/target concentration in the PD medical fluid is typically 1.36%, 2.27%, or 3.86%). The difference between the first target conductivity κ*_{A}* and the initial conductivity κ*_{SSR_init}* is denoted Δκ*_{SSR_init}* Using Equation (1), where the final value *F_{final}* is equal to Δ*κ_{SSR_final},* and the initial value *Fᵢₙᵢₜ* is equal to Δ*K_{SSR_init},* and given that the first target conductivity κ*_{A}* is 12.686 mS/cm, the medical fluid target conductivity κ*_{final}* is 12.35 mS/cm and the overdosed rate *Q_{SSR_init}* was 20.0 ml/min, the final dosing rate of the SSR dosing mechanism 25a becomes: $\begin{matrix}{Q}_{SSR _ final} = \frac{{F}_{final}}{{F}_{init}} ⋅ {Q}_{SSR _ init} = \frac{{Δκ}_{SSR _ final}}{{Δκ}_{SSR _ init}} ⋅ {Q}_{SSR _ init} = \frac{12.686 - 12.35}{12.686 - 11.5} ⋅ 20 \\ = 5.67 ml / min .\end{matrix}$ The SSR-component dosing mechanism 25a is thereafter set to the final dosing rate *Q_{SSR_final}* = 5.67 ml/min. The SSR-component dosing mechanism 25a to main pump 23 speed ratio is locked and stored in memory 50b. For example, if the flow rate of the main pump is 200 ml/min, the speed ratio becomes 200/5.67=35.3. In case the flow rate of the main pump changes, this ratio is used to determine a new flow rate of the SSR-component dosing mechanism 25a. The ratio may be referred to as a dilution ratio of the SSR-component, as has been previously described. Then, in the second phase, follows an optional stabilization period with locked ratio in which the conductivity is allowed to stabilize. This stabilization phase lasts for about 100-200s.

Compared to a direct control of the SSR-component dosing mechanism 25a based on conductivity feedback, this overdose control aligned with steps S1 to S5 gives an improved conductivity signal-to-noise ratio. After the electrolyte component dosing mechanism 24a and the SSR-component dosing mechanism 25a have locked their speeds to the speed of the main pump 23, the mixing system 20 is producing the dialysis medical fluid with the desired composition. Until the accurate and desired composition of the dialysis medical fluid has been reached, the fluid is passed to drain 28. When the correct composition is produced, the first valve 16 is opened and the second valve 17 is closed, so the dialysis medical fluid can be passed to the outlet point 29.

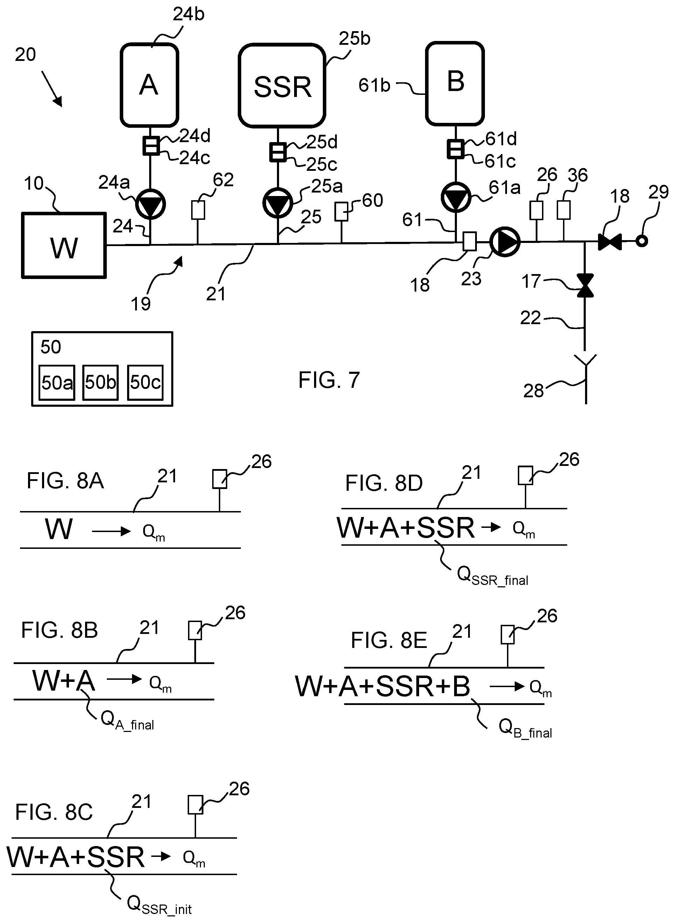

Fig. 7 is a schematic view of an example mixing system 20 according to some embodiments. The mixing system 20 in Fig. 7 is similar to the mixing system in Fig. 2, and reference is made to the description of the mixing system in Fig. 2 for same references. More in detail, the fluid path 19 in the mixing system 20 in Fig. 7 comprises an electrolyte component line 24 and an additional electrolyte component line, 61 and an electrolyte dosing mechanisms 24a and an additional electrolyte dosing mechanisms 61a, with accompanying connectors as have been previously described in connection with Fig. 2. The electrolyte component line 24 is fluidly connected to the main fluid line 21 upstream the connection point of the SSR-component line 25 to the main fluid line 21. The additional electrolyte component line 61 is fluidly connected to the main fluid line 21 downstream the connection point of the SSR-component line 25 to the main fluid line 21, but upstream the main pump 23. Compared to the mixing system in Fig. 2, the mixing system 20 in Fig. 7 comprises a first conductivity sensor 62 configured to sense the conductivity of the fluid in the main fluid line 21 downstream the connection point of the first electrolyte component line 24 to the main fluid line 21, but upstream the connection point of the SSR-component line 25 to the main fluid line 21. The mixing system 20 in Fig. 7 further comprises a second conductivity sensor 60 configured to sense the conductivity of the fluid in the main fluid line 21 downstream the connection point of the SSR-component line 25 to the main fluid line 21, but upstream the connection point of the second electrolyte component line 61 to the main fluid line 21.

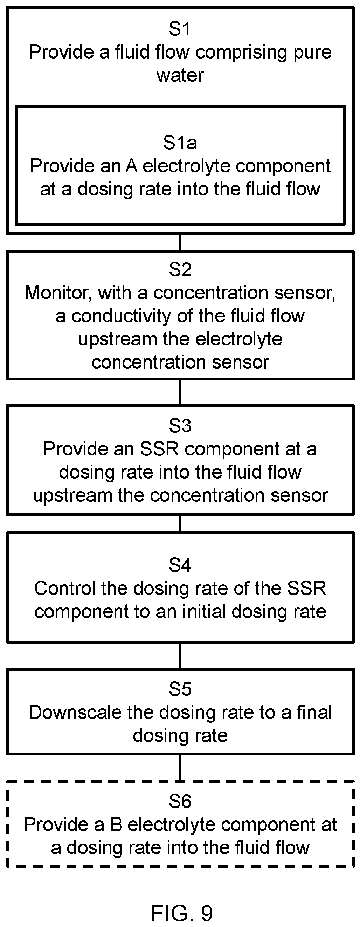

In the following, an example of mixing including dosing of an SSR-component as illustrated in Figs. 8A to 8E, and by the flow chart in Fig. 9, will be explained, that may use the mixing system 20 in Fig. 7. In more detail, the flow chart in Fig. 9 illustrates method steps for mixing components of a medical fluid that can be implemented by the control arrangement 50 in Fig. 7. Hence, the control arrangement 50 is configured to perform all the steps, examples and embodiments outlined below in relation to the Figs. 8A to 9.

Again, in these steps, examples and embodiments, the mixing includes temporarily overdosing the SSR-component to be able to accurately sense the concentration of the SSR-component, and subsequently downscaling the dosing rate of the SSR-component to a final dosing rate, which complies with a prescription including a final concentration of the SSR-component in the final medical fluid. Hence, what is described regarding such overdosing and downscaling in relation to the examples in Figs. 1 to 6, can be equally applied to the examples described in relation to the Figs. 7 to 9.

Figs. 8A to 8E are illustrations of different steps for accurately dosing an SSR-component in the mixing system 20 of Fig. 7 according to some embodiments. The different steps are here explained in a specific order but may in other embodiments have another order. A part of the main fluid line 21 of the mixing system 20 is schematically illustrated in the figures, together with the concentration sensor 26. In this example, the SSR-component is an electrolyte component that is to be dosed in a low amount into a mix of pure water and an additional electrolyte component (A) in the main fluid line 21. Hence, when the SSR-component is added to the mix of pure water and the electrolyte component A in the main fluid line 21, the conductivity of the mixed fluid will increase compared to the conductivity of the mix of pure water and the electrolyte component A. Compared to the example illustrated in Figs. 3A to 3C, the addition of the electrolyte component A before the SSR-component is added will move the range where the conductivity shall be measured to a range where the conductivity can be more accurately measured by the concentration sensor 26. The desired final composition of the medical fluid is predetermined and known, and hence also the intended final concentration of the SSR-component in the medical fluid. The final concentration of the SSR-component in the medical fluid is so small that the continuous dosed flow rate to provide the final concentration of the SSR-component would not give a reliable conductivity measurement with the concentration sensor 26.

With reference to the flow chart in Fig. 9, in a first step S1, a flow of fluid comprising a mixture of pure water (W) and the electrolyte component A is provided in the main fluid line 21, e.g., with a flow rate of *Qₘ* ml/min.

In a second step S2, the conductivity of the fluid is monitored by the concentration sensor 26. The first step S1 and the second step S2 are typically performed simultaneously, hence, while fluid is flowing in the main fluid line 21 the conductivity of the fluid will be monitored. Pure water is provided in the same way as in the example explained with reference to Fig. 3A to 3C and has the same purity. Hence, as illustrated in Fig. 8A, a flow of only pure water W is provided in the main fluid line 21, where the arrow indicates the direction of the flow. As only pure water is flowing in the main fluid line 21, the flow rate provided with the main pump 23 will be equal to the pure water flow rate. Just as in the example in Figs. 4A-4D, the present example comprises also adding an electrolyte component A into the main fluid line 21, before the SSR-component is added. The providing includes providing S1a the electrolyte component A, with an electrolyte component dosing mechanism 24A, at a dosing rate into the flow of pure water in the main fluid line 21 to form the flow of fluid comprising a mixture of pure water and the electrolyte component A upstream the concentration sensor 26. This is illustrated in Fig. 8B, where a dosing rate of the electrolyte component A has been added to the flow of pure water. The pure water flow rate is thereby reduced with the electrolyte component A's dosing rate, so the total flow rate in the main fluid line 21 is still the same, hence *Qₘ* ml/min. With the addition of the electrolyte component A, the conductivity of the fluid has been increased compared to the conductivity of the flow of only pure water. Also, just in the example of Figs. 4A to 4D, the providing may include the step S1a of controlling the dosing rate of the electrolyte component A using conductivity feedback from the concentration sensor 26. This dosing rate is referred to as the final dosing rate *Q_{A_final}* of the electrolyte component A. In some embodiments, the providing S1 comprises fixing a ratio between the final dosing rate *Q_{A_final}* of the electrolyte component and the main flow rate. Thereby, if the main flow rate changes, the dosing rate of the electrolyte component A will also change accordingly to maintain the ratio and thereby the final composition of the medical fluid. The total flow rate in the main fluid line 21 is still the same *Qₘ* ml/min, but the pure water flow rate W has been decreased to: *Qₘ* ml/min minus *Q_{A_final}* ml/min.

The steps S3 to S5 are thereafter performed in the same way as has been described with reference to Figs. 3A to 3C, however briefly described below. Hence, in a third step S3, the SSR-component is provided at a dosing rate, with the SSR dosing mechanism 25a, into the flow of fluid upstream the concentration sensor 26. The fluid flow is thereby reduced with the SSR-component dosing rate, so the total flow rate in the main fluid line 21 is still the same, hence *Qₘ* ml/min.

The dosing rate of the SSR-component is thereafter controlled S4, with the SSR dosing mechanism 25a, to an initial dosing rate *Q_{SSR_init}* ml/min at which the conductivity monitored with the concentration sensor 26 indicates a predetermined initial concentration *c_{SSR_init}* of the SSR-component in the fluid. The initial concentration *c_{SSR_init}* of the SSR-component is greater than an intended final predetermined concentration *c_{SSR_final}* of the SSR-component in the medical fluid, in order to determine a relationship between the dosing rate of the SSR-component and the resulting concentration of the SSR-component in the fluid. This is illustrated in Fig. 8C. As also the SSR-component has been added, the pure water flow rate W is decreased to: *Qₘ* ml/min minus *Q_{SSR_init}* minus *Q_{A_final}* ml/min after step S4 as illustrated in Fig. 8C. For the signal-to-noise ratio, $SNR = \frac{S}{N}$, the signal S is for example the measured conductivity response at the initial dosing rate *Q_{SSR_init},* and the noise quantification N is for example the standard deviation of the measured conductivity response at the final dosing rate *Q_{A_final}* during a time period.

In the further step S5, the method includes downscaling, with the SSR dosing mechanism 25a, the dosing rate of the SSR-component to the final dosing rate *Q_{SSR_final}* at which the final predetermined concentration *c_{SSR_final}* of the SSR-component in the fluid is achieved, based on the determined relationship. The pure water flow rate W is then increased to: *Qₘ* ml/min minus *Q_{SSR_final}* minus *Q_{A_final}* ml/min after step S5 as illustrated in Fig. 8D. Also, the conductivity at the initial dosing rate *Q_{SSR_init}* corresponds to a predetermined target conductivity *Kᵢₙᵢₜ* of the fluid determined also based on the added conductivity from the electrolyte component. After the SSR-component has been dosed with an accurate dosing rate, an additional electrolyte component B may be dosed as illustrated with the step S6 in the flow chart of Fig. 9 and further explained with reference to the example of Figs. 4A to 4D.

In one embodiment, the mixing system 20 in Fig. 7 may, e.g., be a system configured to mix a medical fluid for an EC blood therapy. The SSR-component may in such a case be a potassium concentrate. The electrolyte component A may then be a concentrate comprising an acid and electrolytes such as sodium, calcium and magnesium chloride. It may also contain glucose. The electrolyte component B is in such a system a bicarbonate concentrate. The separate dosing of potassium enables any, within a reasonable range, concentration of potassium in the medical fluid. In some embodiments, the SSR-component comprises potassium with a concentration of 400-3200 mM/l.

In the example, the main pump 23 starts pumping pure water from the water purification system 10 at a predetermined rate, corresponding to step S1. The first electrolyte dosing mechanism 24a thereafter starts dosing the electrolyte component A from the electrolyte component container 24b, corresponding to step S1a. The flow rate of the first electrolyte dosing mechanism 24a is controlled by conductivity feedback from the concentration sensor 26, such that a first target conductivity value κ*_{A}* is reached. Using a master concentration cell/sensor as described herein, i.e., the concentration sensor 26, means all mixing steps are setup using the same cell/sensor. This corresponds to step S2. That further means the following potassium and bicarbonate mixing steps, which add comparably little to the conductivity, are dosed relative to the first target conductivity value κ*_{A}*. This greatly increases the accuracy of the mixing by the mixing system 20. When the pre-determined conductivity value has been reached, the speed of the first electrolyte dosing mechanism 24a at the pre-determined conductivity value is locked to the speed of the main pump 23. Hence, a ratio between the final dosing rate *Q_{A_final}* of the electrolyte component and the main flow rate is fixed. The first electrolyte dosing mechanism 24a and the main pump 23 are for example piston pumps. The first conductivity sensor 62 is at this point "adjusted" (an offset may be added) such that it presents the same reading as the concentration sensor 26. The first conductivity sensor 62 is used for protective measures, to supervise that the dosing of the electrolyte component A will function as intended during the following mixing of the other components. When the speed of the first electrolyte dosing mechanism 24a has been fixed, the SSR-component mechanism 25a starts dosing potassium into the main flow line 21, corresponding to step S3. The conductivity contribution of potassium is small. Hence, the mixing system 20 needs to overdose potassium in an overdosing step, where the SSR-component mechanism 25a is run at a much higher flow rate than the flow rate that would give the desired potassium content in the final medical fluid. This corresponds to step S4. By overdosing potassium in this way, a high enough increase in conductivity at the concentration sensor 26 is accomplished such that it can be accurately measured with the same sensor, i.e., making conductivity control of the component possible. In one example embodiment, the potassium in the SSR-component container 25b has a concentration of 800 mmol/l. This will result in a flow rate of 0.625 ml/min when the main flow rate is 250 ml/min to get a resulting potassium concentration in the dialysis fluid of 2 mmol/l. The set conductivity, when going from adding only electrolyte component A to adding electrolyte component A and potassium, then increases from, in an example calculation, 11.61 to 11.85 mS/cm. This increase cannot be measured accurately with the concentration sensor 26. If on the other hand the potassium flow rate is increased tenfold, i.e., to 6.25 ml/min, the step instead increases from 11.61 to 14.02 mS/cm, which change can be measured accurately with the concentration sensor 26. A predetermined target conductivity κ*_{SSR_init}* corresponding to the increased flow rate of potassium can be calculated, and the SSR dosing mechanism controlled to a dosing rate that corresponds to the predetermined target conductivity, using conductivity feedback, as previously described. Once the predetermined target conductivity value of the fluid has been reached, the control arrangement 50 thereafter uses Equation (1) to relate the actual composition in the potassium container 25b to the dosing rate of the SSR mechanism 25a to get to a desired concentration of potassium in the final medical fluid. The dosing rate of the SSR dosing mechanism 25a is thereafter scaled down to meet that final desired potassium concentration, corresponding to step S5. Alternatively, and as has been previously described, the control arrangement 50 may instead set the SSR dosing mechanism 25a to a fix flow rate *Q_{SSR_init}* such that the resulting conductivity step is sufficiently high. The resulting stabilized conductivity is measured, giving a κ*_{SSR_init}.* The relation between this conductivity and the predetermined target conductivity κ*_{SSR_final},* multiplied with the fix flow rate *Q_{SSR_init}* will then give the final flow rate of the SSR dosing mechanism 25a such that the desired final concentration of the SSR-component is reached. The rotational speed of the SSR mechanism 25a, is, as was the first electrolyte dosing mechanism 24a above, locked to the speed of the main pump 23, hence, a dilution ratio of the SSR-component is established. After the dosing of the electrolyte component A and potassium have been established, the second dosing mechanism 61a starts adding bicarbonate to the fluid in the main fluid line 21. The routine is the same as for the first dosing steps. The target conductivity κ*_{final}* to control against at the concentration sensor 26 is now the one representing the final medical fluid composition. In one embodiment, this conductivity is between 12-16 mS/cm. As what comes out of the second electrolyte component container 61b is, due to temperature, varying in composition it's important the flow of the bicarbonate concentrate is controlled against conductivity at all times during the treatment.

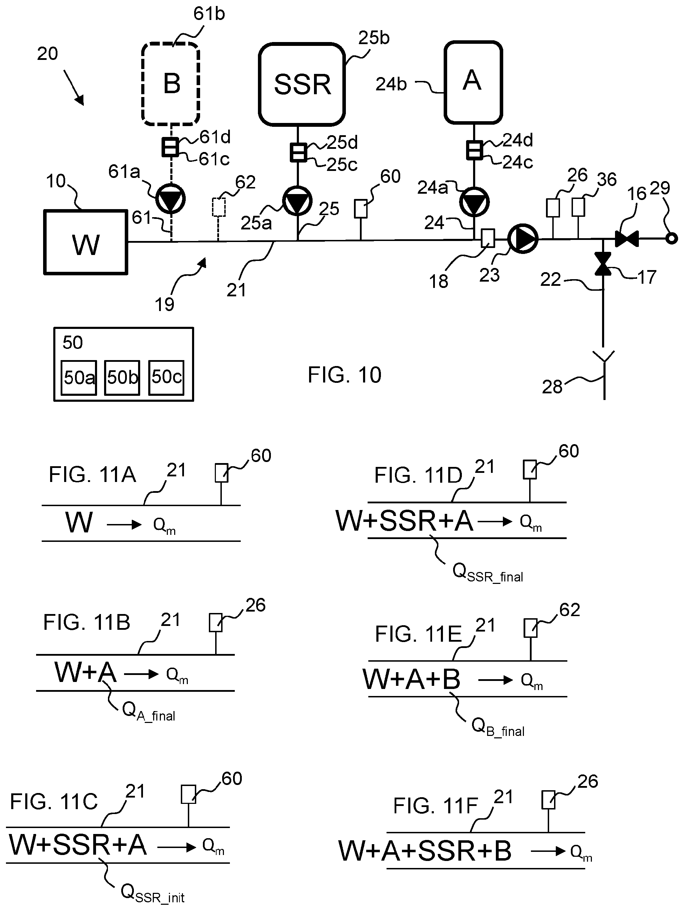

Fig. 10 is illustrating a schematic view of an example mixing system according to some embodiments. The mixing system 20 in Fig. 10 is very similar to the mixing system in Fig. 7, and reference is made to the description of Fig. 7 for explaining the same references. The mixing system 20 in Fig. 10 differs from the mixing system in Fig. 7 in that the components for dosing electrolyte component A has changed place with the components for dosing electrolyte component B. Hence, the electrolyte component A is mixed into the main fluid line 21 downstream the point where the SSR-component is mixed into the main fluid line 21, but upstream the main pump 23. The control arrangement 50 is configured to perform all the steps, examples and embodiments outlined below in relation to the Figs. 11A to 11F.

Again, in these steps, examples and embodiments, the mixing includes temporarily overdosing the SSR-component to be able to accurately sense the concentration of the SSR-component, and subsequently downscaling the dosing rate of the SSR-component to a final dosing rate, which complies with a prescription including a final concentration of the SSR-component in the final medical fluid. Hence, what is described regarding such overdosing and downscaling in relation to the examples in Figs. 1 to 9, can be equally applied to the examples described in relation to the Figs. 10 and 11.

Figs. 11A to 11F are illustrations of different steps for accurately dosing an SSR-component in the mixing system 20 of Fig. 10 according to some embodiments of the disclosure. A part of the main fluid line 21 of the mixing system 20 is schematically illustrated in the figures, together with the first conductivity sensor 62, the second conductivity sensor 62 and the concentration sensor 26. In this example, the SSR-component comprises both conductive components and a non-conductive component. The effect is such that the SSR-component will increase the conductivity of the fluid into which it will be dosed. The SSR-component is to be dosed in a low amount to the main fluid line 21. For example, the SSR-component is a concentrate including potassium (K⁺), calcium (Ca²⁺) and glucose (G). Such SSR-component is for example the concentrate in a SelectBag^{™}, e.g., SelectBag One or Selectbag Citrate, from Baxter. Such SSR-component is typically dosed with a dilution ratio of 1:200. If the main flow rate is 500 ml/min, that means that the SSR-component shall be dosed with approximately 2.5 ml/min, which is a low flow compared to the main flow rate. The electrolyte component A is for example a SelectCart^{™} from Baxter comprising sodium chloride (NaCl) as powder. The electrolyte component B is for example a BiCart^{™} cartridge from Baxter with sodium bicarbonate (NaHCO₃) powder. Out of a BiCart^{™} container, a bicarbonate concentrate with the approximate concentration of 1200 mmol/l is flowing. In one embodiment, the final medical fluid has a composition of sodium 140 mmol/l, bicarbonate 34 mmol/l, kalium 2mM/l, Ca²⁺ 1.5 mM/l, Mg²⁺ 0.5 mM/l, Ac⁻ 3 mM/l, glucose 5.55 mM/l and Cl⁻ 109 mM/l.

In the example, the main pump 23 starts pumping pure water from the water purification system 10 at a predetermined rate *Qₘ* ml/min, as illustrated in Fig. 11A. The first electrolyte dosing mechanism 24a thereafter starts dosing the electrolyte component A from the electrolyte component container 24b as illustrated in Fig. 11B. The flow rate of the first electrolyte dosing mechanism 24a is controlled by conductivity feedback from the concentration sensor 26, such that a target conductivity value κ*_{A}* is reached. When the pre-determined conductivity value has been reached, the dosing rate *Q_{A_final}* of the first electrolyte dosing mechanism 24a at the target conductivity value is locked to the speed of the main pump 23. Hence, a ratio between the final dosing rate *Q_{A_final}* of the electrolyte component and the main flow rate is fixed. The ratio is saved in memory, and the dosing of the electrolyte component A is stopped, to save on concentrate. Thereafter, the SSR-component mechanism 25a starts dosing the SSR-component into the main flow line 21, and thus into the flow of pure water. The conductivity of the mix of the SSR-component and pure water is to be measured with the second conductivity sensor 60. The conductivity contribution of the SSR-component with potassium (K⁺), calcium (Ca²⁺) and glucose (G) is small. However, the second conductivity sensor 60 is to be used as a protective conductivity sensor during continuous mixing and is therefore configured for another conductivity range configured for a fluid comprising electrolyte component B, the SSR-component and pure water. Hence, the mixing system 20 needs to overdose the SSR-component in an "overdosing phase", where the SSR-component mechanism 25a is run at a much higher flow rate than the flow rate that would give the desired SSR-component content in the final medical fluid. By "overdosing" the SSR-component in this way, a high enough increase in conductivity at the second conductivity sensor 60 is accomplished such that conductivity control becomes possible. Once a predetermined target conductivity κ*_{A}* for the mixture of the SSR-component, the electrolyte component A and the pure water, when the SSR-component is overdosed, has been reached as illustrated in Fig. 11C, the mixing system 20 thereafter uses Equation (1) to relate the actual composition in the SSR-component container 25b to the rotational speed of the SSR mechanism 25a to get to a desired concentration of the SSR-component in the final medical fluid. The rotational speed of the SSR mechanism 25a is thereafter scaled down to meet that final desired SSR-component concentration, as illustrated in Fig. 11D. The rotational speed of the SSR mechanism 25a, is, as was the first electrolyte dosing mechanism 24a above, locked to the speed of the main pump 23, resulting in a dosing ratio for the SSR mechanism.

Alternatively, the dosing of the electrolyte component A is continued, instead of being stopped.

After the dosing of the electrolyte component A and SSR-component have been established, the dosing of the SSR-component is stopped, and the second dosing mechanism 61a starts adding bicarbonate concentrate, hence the electrolyte component B, to the fluid in the main fluid line 21 as illustrated in Fig. 11E. If the dosing of the electrolyte component A has been continued, it can also be stopped. Alternatively, the dosing of the electrolyte component A and the dosing of the SSR-component are continued, but then more concentrate will be wasted. The first conductivity sensor 62 is configured to measure the conductivity of the fluid comprising pure water and the bicarbonate. When the predetermined target conductivity value for the mixture of pure water and bicarbonate has been reached, the speed *Q_{B_final}* of the second electrolyte dosing mechanism 61a at the predetermined target conductivity value is locked to the speed of the main pump 23. Hence, a ratio between the final dosing rate *Q_{B_final}* of the electrolyte component and the main flow rate is fixed. The predetermined target conductivity value *K_{LCR_final}* corresponds to a desired final concentration of bicarbonate in the final medical fluid. At this stage, the first conductivity sensor 62 and/or the second conductivity sensor 60 are adjusted, if needed, to ensure that they measure the same level.

After the dosing rates of the electrolyte component A, the SSR-component and the electrolyte component B have been established, the dosing of all the components is started (if not already ongoing) with flow rates according to the determined ratios, and the main concentrate sensor 26 monitors the conductivity of the final medical fluid, as illustrated in Fig. 11F. The first conductivity sensor 62 monitors the fluid comprising only pure water and the electrolyte component B. The second conductivity sensor 60 monitors the fluid comprising only pure water, the electrolyte component B and the SSR-component. All the conductivity measurements shall correspond to predetermined conductivity values. Especially, a difference between the measurement with the first conductivity sensor 62 and the second conductivity sensor 60 shall be constant. If not, an alarm will be generated, and the source of error can be found using the different conductivity measurements.

Alternatively, the concentration of the electrolyte component B may be regulated directly after the concentration of the electrolyte component A is regulated, and the first conductivity sensor 62 and/or the second conductivity sensor 60 are adjusted, if needed, to ensure that they measure the same level. The dosing of the electrolyte component B is thereafter stopped, while the dosing of the electrolyte component A is continued. First thereafter is the SSR-component dosed according to what has been described above in an overdosing phase in a flow of pure water and the electrolyte component A, and an accurate dosing flow rate of the SSR-component determined and set. The dosing of the of electrolyte component B is thereafter started again at the determined ratio. Thereafter the conductivities measured with the conductivity sensors is performed as described in the previous example.

While the invention has been described in connection with what are presently considered to be the most practical and preferred embodiments, it is to be understood that the invention is not to be limited to the disclosed embodiments, but on the contrary, is intended to cover various modifications. The claimed invention is defined by the appended claims.

## Claims

1. A mixing system (20) for in-line mixing of components of a medical fluid having a final predetermined composition of pure water, a small-signal-response component, SSR-component, and at least one electrolyte component; wherein the mixing system (20) comprises:
- a fluid path (19) comprising
a main fluid line (21) arranged to be connected to a source of pure water (10),
an SSR-component line (25) fluidly connected to the main fluid line (21) and provided with an SSR-component line connector (25c) configured to be connected to an SSR-component container (25b),
an electrolyte component line (24) fluidly connected to the main fluid line (21) and provided with an electrolyte component connector (24c) configured to be connected to an electrolyte component container (24b);
- a main pump (23) arranged to the main fluid line (21) to provide a main flow of fluid in the main fluid line (21);
- a concentration sensor (26, 60) arranged to measure a concentration of the fluid in the main fluid line (21);
- an SSR dosing mechanism (25a) arranged to the SSR-component line (25) to provide the SSR-component at a dosing rate into the main fluid line (21) upstream the concentration sensor (26, 60);
- an electrolyte dosing mechanism (24a) arranged to the electrolyte component line (24) to provide an electrolyte component at a dosing rate into the main fluid line (21) upstream the concentration sensor (26, 60);
- a control arrangement (50) configured to:
provide, using the main pump (23), a flow of fluid comprising a mixture of pure water from the source of pure water and the electrolyte component from the electrolyte component container (24b), in the main fluid line (21);
monitor, using the concentration sensor (26, 60), a concentration of the fluid;
and **characterized in that** the control arrangement is further configured to control, using the SSR dosing mechanism (25a), the dosing rate of the SSR-component to an initial dosing rate at which the concentration monitored with the concentration sensor (26, 60) indicates an initial concentration (*c*_{*SSR*_*init*}) of the SSR-component in the fluid that is greater than an intended final predetermined concentration (*c_{SSR_final}*) of the SSR-component in the medical fluid, in order to determine a relationship between the dosing rate of the SSR-component and the resulting concentration of the SSR-component in the fluid;
downscale, using the SSR dosing mechanism (25a), the dosing rate of the SSR-component to a final dosing rate (*Q_{SSR_final}*) at which the final predetermined concentration (*c_{SSR_final}*) of the SSR-component in the fluid is achieved, based on the determined relationship.

2. The mixing system according to claim 1, wherein the initial concentration (*c_{SSR_init}*) of the SSR-component in the fluid is greater than the intended final predetermined concentration (*c_{SSR_final}*) of the SSR-component in the medical fluid such that a ratio between a concentration response resulting from SSR-component dosing at the initial dosing rate (*Q_{SSR_init}*) and concentration signal noise is equal to or greater than a predetermined limit.

3. The mixing system (20) according to claim 1 or 2, wherein the initial dosing rate (*Q_{SSR_init}*) of the SSR-component is > 1 to 20 times larger than the final dosing rate (*Q_{SSR_final}*).

4. The mixing system (20) according to any one of the preceding claims, wherein the SSR-component being either of a non-conductive solution or a conductive solution such that, when added to the flow of fluid in the main flow path at an intended final dosing rate (*Q_{SSR_final}*) at which the final predetermined concentration (*c_{SSR_final}*) of the SSR-component in the fluid is achieved, its contribution to the concentration as monitored with the concentration sensor (26, 60) is too small to be measured with sufficient accuracy.

5. The mixing system (20) according to any one of the preceding claims, wherein the concentration measured with the concentration sensor (26, 60) at the initial dosing rate (*Q_{SSR_init}*) corresponds to a predetermined target concentration (K_{*SSR*_*init*}) of the fluid.

6. The mixing system (20) according to any one of the preceding claims , wherein the initial dosing rate corresponds to a predetermined initial dosing rate (*Q_{SSR_init}*).

7. The mixing system (20) according to any one of the preceding claims, wherein the control arrangement (50) is configured to determine the final dosing rate (*Q*_{*SSR*_*final*}) as being equal to a ratio between a final value (*F_{final}*) correlated with the intended final predetermined concentration (*c_{SSR_final}*) of the SSR-component and an initial value (*Fᵢₙᵢₜᵢₐₗ*) correlated with the initial concentration (*C_{SSR_final}*) of the SSR-component, multiplied with the initial dosing rate (*Q_{SSR_init}*).

8. The mixing system (20) according to any one of the preceding claims, wherein the control arrangement (50) is configured to control the main pump (23) to provide the main flow rate to be a predetermined fluid rate of medical fluid.

9. The mixing system (20) according to any one of the preceding claims, wherein the control arrangement (50) is configured to fix a ratio between the final dosing rate (*Q_{SSR_final}*) of the SSR-component and the main flow rate.

10. The mixing system (20) according to any one of the preceding claims, wherein the concentration sensor (26, 60) is a conductivity sensor.

11. The mixing system (20) according to claim 10, wherein the control arrangement (50) is configured to monitor with the same concentration sensor (26).

12. The mixing system (20) according to any one of the preceding claims, wherein the control arrangement (50) is configured to, preceding the providing of the SSR-component into the flow of fluid, control the dosing rate of the electrolyte component, with the electrolyte component dosing mechanism (24A), to a final dosing rate (*Q_{A_final}*) providing a final predetermined concentration (*c_{A_final}*) of the electrolyte component in the final medical fluid, based on the concentration monitored by the concentration sensor (26) of the mixture of pure water and the electrolyte components.

13. The mixing system (20) according to claim 8, and anyone of claims 10 to 12, wherein the control arrangement (50) is configured to fix a ratio between the final dosing rate (*Q_{A_final}*) of the electrolyte component and the main flow rate.

14. The mixing system (20) according to any one of claims 10 to 13, wherein the SSR-component decreases the conductivity of the fluid into which it is added.

15. The mixing system (20) according to any one of the preceding claims, wherein the SSR-component comprises glucose.

## Patentansprüche

1. Mischsystem (20) zum Inline-Mischen von Komponenten eines medizinischen Fluids mit einer endgültigen vorbestimmten Zusammensetzung aus reinem Wasser, einer Komponente mit geringer Signalreaktion (Small Signal Response component, SSR-Komponente) und wenigstens einer Elektrolytkomponente; wobei das Mischsystem (20) umfasst:
- einen Fluidpfad (19), umfassend
eine Hauptfluidleitung (21), die dazu angeordnet ist, mit einer Quelle reinen Wassers (10) verbunden zu werden, eine SSR-Komponentenleitung (25), die in Fluidverbindung mit der Hauptfluidleitung (21) steht und mit einem SSR-Komponentenleitungsverbinder (25c) ausgestattet ist, der dazu ausgelegt ist, mit einem SSR-Komponentenbehälter (25b) verbunden zu werden,
eine Elektrolytkomponentenleitung (24), die in Fluidverbindung mit der Hauptfluidleitung (21) steht und mit einem Elektrolytkomponentenverbinder (24c) ausgestattet ist, der dazu ausgelegt ist, mit einem Elektrolytkomponentenbehälter (24b) verbunden zu werden;
- eine Hauptpumpe (23), die an der Hauptfluidleitung (21) angeordnet ist, um einen Hauptfluss eines Fluids in der Hauptfluidleitung (21) bereitzustellen;
- einen Konzentrationssensor (26, 60), der dazu angeordnet ist, eine Konzentration des Fluids in der Hauptfluidleitung (21) zu messen;
- einen SSR-Dosiermechanismus (25a), der an der SSR-Komponentenleitung (25) angeordnet ist, um die SSR-Komponente mit einer Dosierrate in die Hauptfluidleitung (21) stromaufwärts des Konzentrationssensors (26, 60) bereitzustellen;
- einen Elektrolytdosiermechanismus (24a), der an der Elektrolytkomponentenleitung (24) angeordnet ist, um eine Elektrolytkomponente mit einer Dosierrate in die Hauptfluidleitung (21) stromaufwärts des Konzentrationssensors (26, 60) bereitzustellen;
- eine Steueranordnung (50), ausgelegt zum:
Bereitstellen, unter Verwendung der Hauptpumpe (23), eines Fluidflusses, umfassend eine Mischung aus reinem Wasser von der Quelle reinen Wassers und der Elektrolytkomponente aus dem Elektrolytkomponentenbehälter (24b), in der Hauptfluidleitung (21);
Überwachen, unter Verwendung des Konzentrationssensors (26, 60), einer Konzentration des Fluids;
und **dadurch gekennzeichnet, dass** die Steueranordnung ferner ausgelegt ist, um unter Verwendung des SSR-Dosiermechanismus (25a) die Dosierrate der SSR-Komponente auf eine anfängliche Dosierrate zu steuern, bei der die Konzentration, die mit dem Konzentrationssensor (26, 60) überwacht wird, eine anfängliche Konzentration (c_{SSR_init}) der SSR-Komponente in dem Fluid angibt, die höher ist als eine vorgesehene endgültige vorbestimmte Konzentration (c_{SSR_final}) der SSR-Komponente in dem medizinischen Fluid, um eine Beziehung zwischen der Dosierrate der SSR-Komponente und der resultierenden Konzentration der SSR-Komponente in dem Fluid zu bestimmen;
Herunterskalieren, unter Verwendung des SSR-Dosiermechanismus (25a), der Dosierrate der SSR-Komponente auf eine endgültige Dosierrate (Q_{SSR_final}), bei der die endgültige vorbestimmte Konzentration (c_{SSR_final}) der SSR-Komponente in dem Fluid erzielt wird, basierend auf der bestimmten Beziehung.

2. Mischsystem gemäß Anspruch 1, wobei die anfängliche Konzentration (c_{SSR_init}) der SSR-Komponente in dem Fluid höher ist als die vorgesehene endgültige vorbestimmte Konzentration (c_{SSR_final}) der SSR-Komponente in dem medizinischen Fluid, sodass ein Verhältnis zwischen einer Konzentrationsreaktion resultierend aus einer SSR-Komponentendosierung mit der anfänglichen Dosierrate (Q_{SSR_init}) und einem Konzentrationssignalrauschen größer oder gleich einem vorbestimmten Grenzwert ist.

3. Mischsystem (20) gemäß Anspruch 1 oder 2, wobei die anfängliche Dosierrate (Q_{SSR_init}) der SSR-Komponente > 1 bis 20 mal höher ist als die endgültige Dosierrate (Q_{SSR_final})

4. Mischsystem (20) gemäß einem der vorstehenden Ansprüche, wobei es sich bei der SSR-Komponente entweder um eine nicht leitfähige Lösung oder eine leitfähige Lösung handelt, sodass, wenn diese zu dem Fluidfluss in dem Hauptflusspfad mit einer vorgesehenen endgültigen Dosierrate (Q_{SSR_final}) hinzugegeben wird, bei der die endgültige vorbestimmte Konzentration (c_{SSR_final}) der SSR-Komponente in dem Fluid erzielt wird, deren Beitrag zu der Konzentration, wie mit dem Konzentrationssensor (26, 60) überwacht, zu klein ist, um mit ausreichender Genauigkeit gemessen zu werden.

5. Mischsystem (20) gemäß einem der vorstehenden Ansprüche, wobei die mit dem Konzentrationssensor (26, 60) gemessene Konzentration bei der anfänglichen Dosierrate (Q_{SSR_init}) einer vorbestimmten Zielkonzentration (K_{SSR_init}) des Fluids entspricht.

6. Mischsystem (20) gemäß einem der vorhergehenden Ansprüche, wobei die anfängliche Dosierrate einer vorbestimmten anfänglichen Dosierrate (Q_{SSR_init}) entspricht.

7. Mischsystem (20) gemäß einem der vorhergehenden Ansprüche, wobei die Steueranordnung (50) ausgelegt ist, um die endgültige Dosierrate (Q_{SSR_final}) als gleich einem Verhältnis zwischen einem endgültigen Wert (F_{final}), der mit der vorgesehenen endgültigen vorbestimmten Konzentration (c_{SSR_final}) der SSR-Komponente korreliert, und einem anfänglichen Wert (Fᵢₙᵢₜᵢₐₗ), der mit der anfänglichen Konzentration (c_{SSR_init}) der SSR-Komponente korreliert, multipliziert mit der anfänglichen Dosierrate (Q_{SSR_init}) zu bestimmen.

8. Mischsystem (20) gemäß einem der vorhergehenden Ansprüche, wobei die Steueranordnung (50) dazu ausgelegt ist, die Hauptpumpe (23) zu steuern, um die Hauptflussrate als eine vorbestimmte Fluidrate von medizinischem Fluid bereitzustellen.

9. Mischsystem (20) gemäß einem der vorhergehenden Ansprüche, wobei die Steueranordnung (50) dazu ausgelegt ist, ein Verhältnis zwischen der endgültigen Dosierrate (Q_{SSR_final}) der SSR-Komponente und der Hauptflussrate fest einzustellen.

10. Mischsystem (20) gemäß einem der vorstehenden Ansprüche, wobei der Konzentrationssensor (26, 60) ein Leitfähigkeitssensor ist.

11. Mischsystem (20) gemäß Anspruch 10, wobei die Steueranordnung (50) dazu ausgelegt ist, mit demselben Konzentrationssensor (26) zu überwachen.

12. Mischsystem (20) gemäß einem der vorhergehenden Ansprüche, wobei die Steueranordnung (50) ausgelegt ist, um vor dem Bereitstellen der SSR-Komponente in den Fluidfluss basierend auf der Konzentration, die durch den Konzentrationssensor (26) der Mischung aus reinem Wasser und den Elektrolytkomponenten überwacht wird, die Dosierrate der Elektrolytkomponente mit dem Elektrolytkomponentendosiermechanismus (24A) auf eine endgültige Dosierrate (Q_{A_final}) zu steuern, die eine endgültige vorbestimmte Konzentration (c_{A_final}) der Elektrolytkomponente in dem endgültigen medizinischen Fluid bereitstellt.

13. Mischsystem (20) gemäß Anspruch 8 und einem der Ansprüche 10 bis 12, wobei die Steueranordnung (50) dazu ausgelegt ist, ein Verhältnis zwischen der endgültigen Dosierrate (Q_{A_final}) der Elektrolytkomponente und der Hauptflussrate fest einzustellen.

14. Mischsystem (20) gemäß einem der Ansprüche 10 bis 13, wobei die SSR-Komponente die Leitfähigkeit des Fluids, dem sie zugegeben wird, senkt.

15. Mischsystem (20) gemäß einem der vorhergehenden Ansprüche, wobei die SSR-Komponente Glucose umfasst.

## Revendications

1. Système de mélange (20) permettant de mélanger en ligne des composants d'un fluide médical ayant une composition prédéfinie finale d'eau pure, un composant de réponse en petits signaux, composant SSR, et au moins un composant électrolytique ; dans lequel le système de mélange (20) comprend :
- un chemin de fluide (19) comprenant
une ligne de fluide principal (21) conçue pour être raccordée à une source d'eau pure (10),
une ligne de composant SSR (25) raccordée fluidiquement à la ligne de fluide principal (21) et dotée d'un raccord de ligne de composant SSR (25c) configuré pour être raccordé à un récipient de composant SSR (25b),
une ligne de composant électrolytique (24) raccordée fluidiquement à la ligne de fluide principal (21) et dotée d'un raccord de composant électrolytique (24c) configuré pour être raccordé à un récipient de composant électrolytique (24b) ;
- une pompe principale (23) agencée par rapport à la ligne de fluide principal (21) pour fournir un flux principal de fluide dans la ligne de fluide principal (21) ;
- un capteur de concentration (26, 60) agencé pour mesurer une concentration du fluide dans la ligne de fluide principal (21) ;
- un mécanisme de dosage SSR (25a) agencé par rapport à la ligne de composant SSR (25) pour fournir le composant SSR à un taux de dosage dans la ligne de fluide principal (21) en amont du capteur de concentration (26, 60) ;
- un mécanisme de dosage d'électrolyte (24a) agencé par rapport à la ligne de composant électrolytique (24) pour fournir un composant électrolytique à un taux de dosage dans la ligne de fluide principal (21) en amont du capteur de concentration (26, 60) ;
- un agencement de commande (50) configuré pour :
fournir, à l'aide de la pompe principale (23), un flux de fluide comprenant un mélange d'eau pure provenant de la source d'eau pure et du composant électrolytique provenant du récipient de composant électrolytique (24b), dans la ligne de fluide principal (21) ;
surveiller, à l'aide du capteur de concentration (26, 60), une concentration du fluide ;
et **caractérisé en ce que** l'agencement de commande est en outre configuré pour commander, en utilisant le mécanisme de dosage SSR (25a), le taux de dosage du composant SSR à un taux de dosage initial auquel la concentration surveillée avec le capteur de concentration (26, 60) indique une concentration initiale (c_{SSR_init}) du composant SSR dans le fluide qui est supérieure à une concentration prédéfinie finale prévue (c_{SSR_final}) du composant SSR dans le fluide médical, afin de déterminer une relation entre le taux de dosage du composant SSR et la concentration ainsi obtenue du composant SSR dans le fluide ;
réduire, en utilisant le mécanisme de dosage SSR (25a), le taux de dosage du composant SSR à un taux de dosage final (Q_{SSR_final}) auquel est atteinte la concentration prédéfinie finale (c_{SSR_final}) du composant SSR dans le fluide, sur la base de la relation déterminée.

2. Système de mélange selon la revendication 1, dans lequel la concentration initiale (c_{SSR_init}) du composant SSR dans le fluide est supérieure à la concentration prédéfinie finale prévue (c_{SSR_final}) du composant SSR dans le fluide médical de telle sorte qu'un rapport entre une réponse de concentration obtenue du dosage du composant SSR au taux de dosage initial (Q_{SSR_init}) et un bruit de signal de concentration soit égal ou supérieur à une limite prédéfinie.

3. Système de mélange (20) selon la revendication 1 ou 2, dans lequel le taux de dosage initial (Q_{SSR_init}) du composant SSR est > 1 à 20 fois plus grand que le taux de dosage final (Q_{SSR_final}).

4. Système de mélange (20) selon l'une quelconque des revendications précédentes, dans lequel le composant SSR est soit une solution non conductrice, soit une solution conductrice de telle sorte que, lorsqu'il est ajouté au flux de fluide dans la voie d'écoulement principal à un taux de dosage final prévu (Q_{SSR_final}) auquel est atteinte la concentration finale prédéfinie (c_{SSR_final}) du composant SSR dans le fluide, sa contribution à la concentration surveillée avec le capteur de concentration (26, 60) soit trop faible pour être mesurée avec une précision suffisante.

5. Système de mélange (20) selon l'une quelconque des revendications précédentes, dans lequel la concentration mesurée avec le capteur de concentration (26, 60) au taux de dosage initial (Q_{SSR_init}) correspond à une concentration cible prédéfinie (K_{SSR_init}) du fluide.

6. Système de mélange (20) selon l'une quelconque des revendications précédentes, dans lequel le taux de dosage initial correspond à un taux de dosage initial prédéterminé (Q_{SSR_init}).

7. Système de mélange (20) selon l'une quelconque des revendications précédentes, dans lequel l'agencement de commande (50) est configuré pour déterminer le taux de dosage final (Q_{SSR_final}) comme étant égal à un rapport entre une valeur finale (F_{final}) corrélée à la concentration prédéfinie finale prévue (c_{SSR_final}) du composant SSR et une valeur initiale (Fᵢₙᵢₜᵢₐₗ) corrélée à la concentration initiale (c_{SSR_final}) du composant SSR, multiplié par le taux de dosage initial (Q_{SSR_init}).

8. Système de mélange (20) selon l'une quelconque des revendications précédentes, dans lequel l'agencement de commande (50) est configuré pour commander la pompe principale (23) pour fournir le débit principal pour être un débit de fluide prédéterminé de fluide médical.

9. Système de mélange (20) selon l'une quelconque des revendications précédentes, dans lequel l'agencement de commande (50) est configuré pour fixer un rapport entre le taux de dosage final (Q_{SSR_final}) du composant SSR et le débit principal.

10. Système de mélange (20) selon l'une quelconque des revendications précédentes, dans lequel le capteur de concentration (26, 60) est un capteur de conductivité.

11. Système de mélange (20) selon la revendication 10, dans lequel l'agencement de commande (50) est configuré pour surveiller avec le même capteur de concentration (26).

12. Système de mélange (20) selon l'une quelconque des revendications précédentes, dans lequel l'agencement de commande (50) est configuré pour, avant la fourniture du composant SSR dans le flux de fluide, commander le taux de dosage du composant électrolytique, avec le mécanisme de dosage de composant électrolytique (24A), à un taux de dosage final (Q_{A_final}) fournissant une concentration finale prédéfinie (c_{A_final}) du composant électrolytique dans le fluide médical final, sur la base de la concentration surveillée par le capteur de concentration (26) du mélange d'eau pure et des composants électrolytiques.

13. Système de mélange (20) selon la revendication 8, et l'une quelconque des revendications 10 à 12, dans lequel l'agencement de commande (50) est configuré pour fixer un rapport entre le taux de dosage final (Q_{A_final}) du composant électrolytique et le débit principal.

14. Système de mélange (20) selon l'une quelconque des revendications 10 à 13, dans lequel le composant SSR diminue la conductivité du fluide dans lequel il est ajouté.

15. Système de mélange (20) selon l'une quelconque des revendications précédentes, dans lequel le composant SSR comprend du glucose.
